(19) Europäisches Patentamt
European Patent Office
Office européen des brevets

(11) Veröffentlichungsnummer : **0 539 329 A1**

# (12) EUROPÄISCHE PATENTANMELDUNG

(21) Anmeldenummer : **92810795.2**

(22) Anmeldetag : **16.10.92**

(51) Int. Cl.[5] : **C07D 209/24,** C07D 209/18, A61K 31/40

(30) Priorität : **25.10.91 CH 3135/91**

(43) Veröffentlichungstag der Anmeldung : **28.04.93 Patentblatt 93/17**

(84) Benannte Vertragsstaaten : **AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Gerspacher, Marc, Dr.**
**Ringstrasse 150**
**CH-4556 Aeschi (CH)**
Erfinder : **Sallmann, Alfred, Dr.**
**Joachimsackerstrasse 12**
**CH-4103 Bottmingen (CH)**
Erfinder : **Schurter, Rolf, Dr.**
**Holzmattstrasse 45**
**CH-4102 Binningen (CH)**

## (54) Acetylen-Verbindungen, verwendbar als Leukotrien-Antagonisten.

(57) Die Erfindung, betrifft Verbindungen der Formel I,

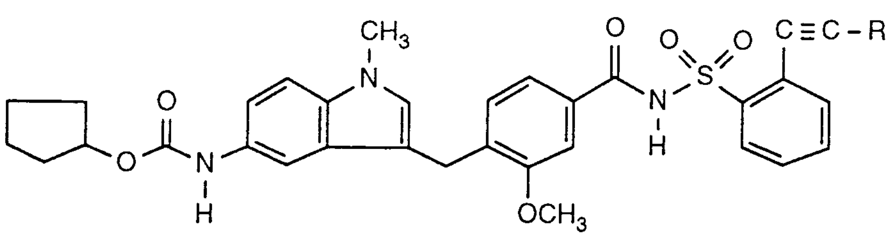

(I)

worin R Wasserstoff oder $C_1$-$C_7$-Alkyl bedeutet oder worin R für das Strukturelement -alk-$R_1$ steht, wobei alk $C_1$-$C_7$-Alkylen, $C_2$-$C_7$-Alkyliden oder $C_3$-$C_6$-Cycloalkyliden bedeutet und $R_1$ für Hydroxy, $C_1$-$C_7$-Alkoxy, Phenyl-$C_1$-$C_7$-alkoxy oder $C_2$-$C_7$-Alkanoyloxy steht ; in freier Form oder in Form eines Salzes ; Verfahren zur ihrer Herstellung, ihre Verwendung sowie pharmazeutische Präparate enthaltend eine Verbindung der Formel I in freier Form oder in Form eines pharmazeutisch verwendbaren Salzes.

EP 0 539 329 A1

Die Erfindung betrifft Verbindungen der Formel I,

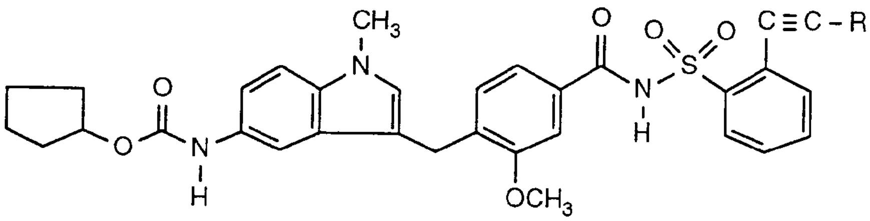

(I)

worin R Wasserstoff oder $C_1$-$C_7$-Alkyl bedeutet oder worin R für das Strukturelement -alk-$R_1$ steht, wobei alk $C_1$-$C_7$-Alkylen, $C_2$-$C_7$-Alkyliden oder $C_3$-$C_6$-Cycloalkyliden bedeutet und $R_1$ Hydroxy, $C_1$-$C_7$-Alkoxy, Phenyl-$C_1$-$C_7$-alkoxy oder $C_2$-$C_7$-Alkanoyloxy bedeutet; in freier Form oder in Form eines Salzes; Verfahren zur ihrer Herstellung, ihre Verwendung sowie pharmazeutische Präparate enthaltend eine Verbindung der Formel I in freier Form oder in Form eines pharmazeutisch verwendbaren Salzes.

Salze von Verbindungen I sind insbesondere pharmazeutisch verwendbare Salze, z. B. Säureadditionssalze, die beispielsweise mit starken anorganischen Säuren, wie Mineralsäuren, z. B. Schwefelsäure, einer Phosphorsäure oder einer Halogenwasserstoftsäure, mit starken organischen Carbonsäuren, wie Niederalkancarbonsäuren, z. B. Essigsäure, wie gegebenenfalls ungesättigten Dicarbonsäuren, z. B. Malon-, Malein- oder Fumarsäure, oder wie Hydroxycarbonsäuren, z. B. Wein- oder Zitronensäure, oder mit Sulfonsäuren, wie Niederalkan- oder gegebenenfalls substituierten Benzolsulfonsäuren, z. B. Methan- oder p-Toluolsulfonsäure, gebildet werden, oder Salze mit Basen, wie entsprechende Alkalimetall- oder Erdalkalimetallsalze, z. B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z. B. Mono-, Di- oder Trihydroxyniederalkylaminen, Hydroxyniederalkyl-niederalkylaminen oder Polyhydroxyniederalkylaminen. Cyclische Amine sind z. B. Morpholin, Thiomorpholin, Piperidin oder Pyrrolidin. Als Mononiederalkylamine kommen beispielsweise Ethyl- und t-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z. B. Mono-, Di- und Triethanolamin; Hydroxyniederalkyl-niederalkyl-amine sind z. B. N,N-Dimethylamino- und N,N-Diethylamino-ethanol; als Polyhydroxyniederalkylamin kommt z. B. Glucosamin in Frage. Umfasst sind ferner für pharmazeutische Verwendungen ungeeignete Salze, da diese beispielsweise für die Isolierung bzw. Reinigung von freien Verbindungen I sowie von deren pharmazeutisch verwendbaren Salzen verwendet werden können.

Vor- und nachstehend sind unter mit "Nieder" bezeichneten Resten und Verbindungen, sofern nicht abweichend definiert, solche zu verstehen, die bis und mit 7, vor allem bis und mit 4, C-Atome aufweisen.

$C_1$-$C_7$-Alkyl ist z.B. Methyl, Ethyl, n-Propyl, Isopropyl, n-Butyl, Isobutyl, sek.-Butyl, tert.-Butyl oder ein entsprechender Pentyl-, Hexyl- oder Heptylrest. Bevorzugt ist $C_1$-$C_5$-Alkyl.

$C_1$-$C_7$-Alkylen ist geradkettig oder verzweigt und bedeutet insbesondere Methylen, Ethylen, 1,2- oder 1,3-Propylen, 1,2-, 1,3-, 1,4- oder 2,3-Butylen, 2-Methyl- 1,2- oder1,3-propylen oder 2,2-Dimethyl-1,2- oder -1,3-propylen. Bevorzugt ist $C_1$-$C_5$-Alkylen.

$C_2$-$C_7$-Alkyliden ist z.B. Ethyliden, 1,1- oder 2,2-Propyliden, 1,1- oder 2,2-Butyliden, 1,1- oder 2,2- oder 3,3-Pentyliden, 2-Methyl-1,1- oder -3,3-butyliden, 2-Methyl- 1,1- oder -3,3-pentyliden.

$C_3$-$C_6$-Cycloalkyliden ist z.B. Cyclopropyliden, Cyclobutyliden, Cyclopentyliden oder Cyclohexyliden. Bevorzugt sind Cyclopentyliden und Cyclohexyliden.

$C_1$-$C_7$-Alkoxy ist z.B. Methoxy, Ethoxy, n-Propyloxy, Isopropyloxy, n-Butyloxy, Isobutyloxy, sek.-Butyloxy, tert.-Butyloxy oder entsprechendes Pentyloxy, Hexyloxy oder Heptyloxy. Bevorzugt ist $C_1$-$C_4$-Alkoxy.

Phenyl-$C_1$-$C_7$-alkoxy ist insbesondere Phenyl-$C_1$-$C_4$-alkoxy und bedeutet vorzugsweise Benzyloxy oder 1- oder 2-Phenethoxy.

$C_2$-$C_7$-Alkanoyloxy ist z.B. Acetyl-, Propionyl-, Butyryl-, Isobutyryl- oder Pivaloyloxy. Bevorzugt ist $C_2$-$C_5$-Alkanoyloxy.

Die Verbindungen I und ihre pharmazeutisch verwendbaren Salze weisen beispielsweise wertvolle pharmakologische Eigenschaften, insbesondere eine ausgeprägte antagonistische Wirkung gegenüber Leukotrienen, auf.

So hemmen sie z.B. in vitro in einem Konzentrationsbereich von etwa 0,001 bis etwa 1 μmol/l die durch Leukotrien $D_4$ ($LTD_4$) induzierte Kontraktion eines glatten Muskels. Diese als $LTD_4$-Antagonismus bezeichnete Wirkung kann experimentell z.B. verifiziert werden, indem in Segmenten, die dem Ileum eines 300 bis 400 g

schweren Meerschweinchens entnommen und in einem Organbad in Tyrode-Lösung bei 38°C und unter Begasung mit einem Gemisch aus 95 % Sauerstoff und 5 % Kohlendioxid bei einer Belastung von 1 g inkubiert wurden, mit synthetischem Leukotrien $D_4$ (in Form des Kaliumsalzes) Kontraktionen ausgelöst und diese isotonisch registriert werden. Das Ausmaß der Hemmung der Kontraktionen durch die Prüfsubstanz wird nach einer Vorinkubation von 2 Minuten in Form der $IC_{50}$ ermittelt, wobei als $IC_{50}$ diejenige Konzentration bezeichnet wird, bei welcher die Testkontraktionen um 50 % reduziert sind

Die Verbindungen I und ihre pharmazeutisch verwendbaren Salze sind auch in vivo ausgezeichnet wirksam. So kann z. B. im Bronchokonstriktions-Standardtest am Meerschweinchen bei Verabreichung einer Aerosollösung, enthaltend von etwa 0,00001 bis etwa 1 Gewichtsprozent Prüfsubstanz, ein deutlicher $LTD_4$-antagonistischer Effekt beobachtet werden. In diesem Testmodell anästhetisiert man männliche, 400 bis 700 g schwere, Meerschweinchen intraperitoneal mit 1,4 g/kg Urethan und führt eine Polyethylenkanüle in die Vena jugularis ein. Eine zweite Polyethylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine luftdicht verschließbare Plexiglaskammer gelegt, die mit einer Fleisch'schen Röhre Nr. 000 und einem Validyne-Transducer MP 45-1 verbunden ist. Mit dieser Anordnung wird der Flow gemessen. Nach der chirurgischen Präparierung der Versuchstiere wartet man eine gewisse Zeit, damit sich die pulmonalen Funktionen stabilisieren können. Die Prüfsubstanz wird anschließend gemäss dem nachfolgenden Protokoll verabreicht. Die Versuchstiere werden während einer Minute einer einprozentigen (Gewicht/Volumen) Aerosollösung der Prüfsubstanz oder destilliertem Wasser (zu Kontrollzwecken) ausgesetzt. Für alle Testverbindungen, welche durch Inhalation verabreicht werden, verwendet man ein Monaghan-Ultraschall-Sprühgerät (Modell 670), dessen Partikelgrösse sich zwischen 1 und 8 Mikron, mit einem Hauptanteil von 3 Mikron, bewegt. Wässrige Lösungen bzw. DMSO/Wasser-Gemische werden jeweils frisch hergestellt und mit einem on-stream drug vial in die Kammer des Sprühgeräts eingeführt. Der produzierte Sprühnebel wird den Versuchstieren über eine Glaskammer von 65 ml Inhalt, die mit einer Kanüle mit der Trachea verbunden wird, verabreicht. Nach Ablauf der Behandlungszeit wird mit einem zweiten Monaghan-Ultraschall-Sprühgerät (Modell 670) und über eine gleiche Glaskammer $LTD_4$ (0,3 µg/ml) während 2 Minuten verabreicht. Es wird die Abnahme der Compliance in der 3. Minute nach $LTD_4$-Applikation abgelesen, und zwar wird der Mittelwert von drei Tieren mit dem Mittelwert von drei Kontrolltieren verglichen und die prozentuale Hemmung der Compliance (% Hemmung) nach folgender Formel errechnet:

$$\% \text{ Hemmung} = 100 - \frac{(100 - \text{Compliance Präparat}) \cdot 100}{(100 - \text{Compliance Kontrolle})}$$

Werden unterschiedliche Wirkstoffkonzentrationen untersucht, so wird die prozentuale Hemmung für jede Konzentration aufgezeichnet, und zwar wird "log Konzentration" auf der Abszisse gegen "prozentuale Hemmung" auf der Ordinate aufgetragen. Die $IC_{50}$ wird dann durch lineare Regressionsanalyse ermittelt.

Die Verbindungen I zeigen ferner eine exzellente Wirkung im von W.H. Anderson et al., Br. J. Pharmacol. 78 (1983) 67, beschriebenen in vivo Test, bei dem durch das Antigen Ovalbumin bei anästhetisierten, künstlich beatmeten Meerschweinchen ein Leukotrienabhängiger Bronchospasmus induziert wird.

Die Verbindungen I und ihre pharmazeutisch verwendbaren Salze weisen weiterhin den spezifischen und therapeutisch sehr bedeutsamen Vorteil einer unerwartet hohen Wirkungsaktivität sowie einer verhältnismäßig langen Wirkungsdauer auf.

Die Verbindungen I und ihre pharmazeutisch verwendbaren Salze können daher überall dort therapeutische Anwendung finden, wo die Wirkung der Leukotriene zu krankhaften Zuständen führt, und diese mildem oder beheben. Die Leukotriene spielen eine wichtige Rolle u. a. bei der Entstehung von allergischen und entzündlichen Prozessen. Demzufolge können die Verbindungen I und ihre pharmazeutisch verwendbaren Salze beispielsweise als Wirkstoffe in Antiallergika verwendet werden, welche z. B. zur Behandlung von allergischen Zuständen und Erkrankungen, wie insbesondere von Asthma, aber auch von Heufieber sowie von obstruktiven Lungenkrankheiten eingesetzt werden. Die Verbindungen der Formel I und ihre pharmazeutisch verwendbaren Salze können z.B. auch zur Behandlung von entzündlichen Erkrankungen der Lunge und anderer Organe verwendet werden, wie der zystischen Fibrose und der Schocklunge (Adult Respiratory Distress Syndrome), aber auch z.B. der Colitis ulcerosa und dem Morbus Crohn, dem septischen Schock und entzündlichen Erkrankungen des Auges.

Ein Gegenstand der Erfindung ist somit die Verwendung der Verbindungen I und ihrer pharmazeutisch verwendbaren Salze zur Herstellung von entsprechenden Arzneimitteln. Dabei ist die gewerbsmäßige Herrichtung der Wirksubstanzen eingeschlossen.

Insbesondere bevorzugt sind Verbindungen der Formel I, worin worin R Wasserstoff oder $C_1$-$C_5$-Alkyl bedeutet oder worin R für das Strukturelement -alk-$R_1$ steht, wobei alk $C_1$-$C_5$-Alkylen, $C_2$-$C_7$-Alkyliden oder $C_3$-$C_6$-Cycloalkyliden bedeutet; und $R_1$ Hydroxy, $C_1$-$C_4$-Alkoxy, Phenyl-$C_1$-$C_4$-alkoxy oder $C_2$-$C_7$-Alkanoyloxy bedeutet; in freier Form oder in Form eines Salzes.

Insbesondere bevorzugt sind Verbindungen der Formel I, worin worin R Wasserstoff oder $C_1$-$C_4$-Alkyl bedeutet, oder worin R für das Strukturelement -alk-$R_1$ steht, wobei alk $C_1$-$C_4$-Alkylen oder $C_2$-$C_5$-Alkyliden bedeutet, und $R_1$ Hydroxy, $C_1$-$C_4$-Alkoxy oder $C_2$-$C_5$-Alkanoyloxy bedeutet; in freier Form oder in Form eines Salzes.

Insbesondere bevorzugt sind Verbindungen der Formel I, worin worin R Wasserstoff oder $C_1$-$C_3$-Alkyl, wie n-Propyl, bedeutet; in freier Form oder in Form eines Salzes.

Insbesondere bevorzugt sind Verbindungen der Formel I, worin R für das Strukturelement -alk-$R_1$ steht; wobei alk $C_1$-$C_4$-Alkylen, wie 1,3-Propylen, oder $C_2$-$C_5$-Alkyliden, wie Ethyliden oder 2,2-Propyliden, bedeutet; und $R_1$ Hydroxy bedeutet; in freier Form oder in Form eines Salzes.

Insbesondere bevorzugt sind Verbindungen der Formel I, worin R für das Strukturelement -alk-$R_1$ steht; wobei alk 1,3-Propylen, Ethyliden, 2,2-Propyliden bedeutet und $R_1$ Hydroxy bedeutet; in freier Form oder in Form eines Salzes.

Namentlich bevorzugt sind im Rahmen der Erfindung die in den Beispielen genannten Verbindungen der Formel I und ihre Salze.

Ebenfalls Gegenstand der vorliegenden Erfindung sind Verfahren zur Herstellung von Verbindungen der Formel I, oder Salzen davon, welche z.B. dadurch gekennzeichnet sind, dass man

a) eine Verbindung der Formel IIa,

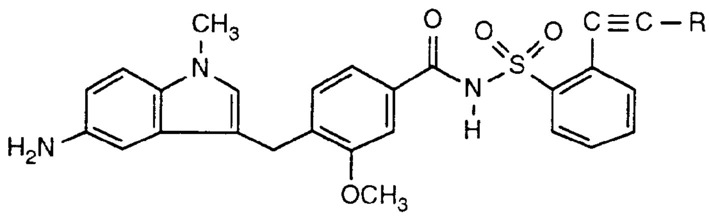

(IIa)

oder ein Salz davon, mit einer Verbindung der Formel IIb,

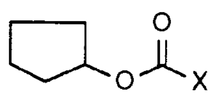

(IIb)

worin X eine nucleofuge Abgangsgruppe ist, unter Abspaltung einer Verbindung H-X umsetzt; oder

b) in einer Verbindung der Formel III,

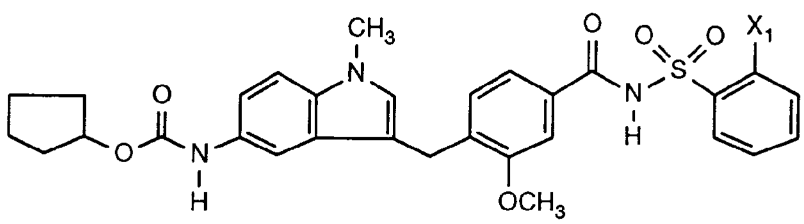

(III)

worin $X_1$ eine in den Rest -C≡C-R überführbare Gruppe bedeutet, oder einem Salz davon, $X_1$ in den Rest -C≡C-R überführt; oder

c) eine Verbindung der Formel IVa,

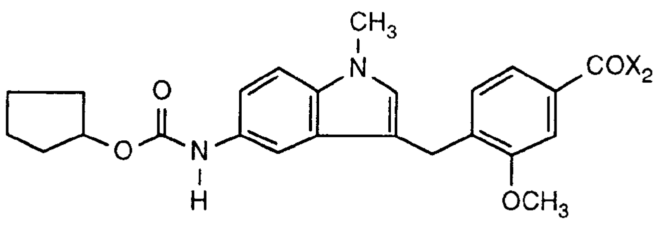

(IVa)

worin $X_2$ für

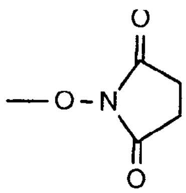 (IVa'), 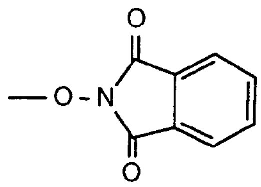 (IVa'')

oder

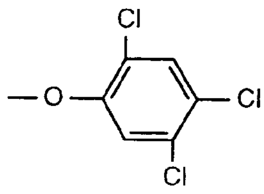 (IVa''')

steht, mit einer Verbindung der Formel IVb,

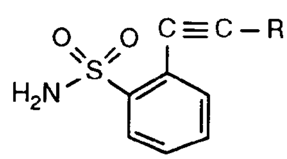 (IVb)

oder einem Salz davon, umsetzt;

und gewünschtenfalls jeweils eine Verbindung der Formel I in eine andere Verbindung der Formel I überführt und/oder eine verfahrensgemäss erhältliche freie Verbindung I in ein Salz überführt oder ein verfahrensgemäss erhältliches Salz einer Verbindung I in die freie Verbindung I oder in ein anderes Salz umwandelt und/oder die Verbindung der Formel I oder ein Salz davon isoliert.

Die vor- und nachstehend beschriebenen Umsetzungen werden in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80°C bis zur Siedetemperatur des Reaktionsmediums, vorzugsweise von etwa -20° bis etwa + 150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Das vor- und nachstehend aufgeführte Ausgangsmaterial, das für die Herstellung der Verbindungen I und ihrer Salze verwendet wird, ist bekannt oder kann nach an sich bekannten Methoden, z.B. gemäss den nachstehend beschriebenen Verfahrensweisen, hergestellt werden.

Für Salze von vor- und nachstehend aufgeführten Ausgangsmaterialien gilt das vorstehend für Salze von Verbindungen I Gesagte in analoger Weise.

Nucleofuge Abgangsgruppen X sind beispielsweise verestertes Hydroxy oder Cyclopentyloxycarbonyloxy.

Eine in den Rest -C≡C-R überführbare Gruppe $X_1$ bedeutet insbesondere Halogen; $X_1$ steht ferner, für die Herstellung von Verbindungen der Formel I, worin R Wasserstoff ist, für eine Gruppe der Formel -C≡C-Z, worin Z z.B. einen Silylrest bedeutet.

Variante a):

Verestertes Hydroxy X ist insbesondere mit einer Mineralsäure verestertes Hydroxy, vor allem Halogen. Bevorzugtes X ist Halogen, wie Chlor, Brom oder Iod. Eine besonders bevorzugte Abgangsgruppe X ist Cyclopentyloxycarbonyloxy.

Die Umsetzung einer Verbindung IIa oder eines Salzes davon mit einer Verbindung IIb erfolgt in üblicher Weise, z. B. unter Kühlen, bei Raumtemperatur oder unter Erwärmen, gegebenenfalls in Gegenwart eines inerten Lösungs- oder Verdünnungsmittels oder eines Gemisches derselben, gegebenenfalls in Gegenwart eines basischen Mittels und/oder unter Inertgas.

Als inerte Lösungs- oder Verdünnungsmittel sind beispielsweise cyclische Ether, aromatische Kohlenwasserstoffe, N,N-Diniederalkylniederalkansäureamide, Phosphorsäureniederalkylamide, Diniederalkylsulfoxide, cyclische Amine und insbesondere gegebenenfalls halogenierte Kohlenwasserstoffe zu nennen, beispielsweise Tetrahydrofuran, Dioxan, Benzol, Toluol, Xylol, N,N-Dimethylformamid, Hexamethylphosphorsäuretriamid, Dimethylsulfoxid, Pyridin, N-Methylmorpholin und insbesondere Hexan und Di- und Trichlormethan.

Geeignete basische Mittel sind z. B. Alkalimetall- oder Erdalkalimetall-hydroxide, -hydride, -amide, -niederalkanolate, -carbonate, -diniederalkylamide oder -niederalkylsilylamide, Niederalkylamine, gegebenenfalls N-niederalkylierte Cycloalkylamine, basische Heterocyclen, Ammoniumhydroxide sowie carbocyclische Amine. Beispielhaft seien Natrium-hydroxid, -hydrid, -amid, -methanolat, Kalium-tert.-butanolat, -carbonat, Lithiumdiisopropylamid, Kalium-bis(trimethylsilyl)-amid, Calciumhydrid, Triethylamin, Cyclohexylamin, N-Cyclohexyl-N,N-dimethyl-amin, Pyridin, N-Methylmorpholin, Benzyl-trimethyl-ammoniumhydroxid sowie 1,8-Diazabicyclo[5.4.0]undec-7-en (DBU) genannt.

In einer bevorzugten Ausführungsform der Variante a) wird eine Verbindung IIa bei Raumtemperatur, unter Stickstoff oder Argon, in einem halogenierten Kohlenwasserstoff, vorzugsweise in Dichlormethan, und in Gegenwart eines basischen Heterocyclus, vorzugsweise in Gegenwart von N-Methylmorpholin, mit einer Verbindung IIb, worin X Halogen, vorzugsweise Chlor, ist, umgesetzt.

In einer besonders bevorzugten Ausführungsform dieser Variante wird die betreffende Verbindung der Formel IIa mit der Verbindung der Formel IIc

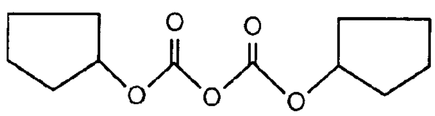

(IIc)

(Dicyclopentyldicarbonat) umgesetzt. Dabei werden verglichen mit dem Einsatz anderer Verbindungen der Formel IIb, beispielsweise solchen, worin X Halogen, wie Chlor ist, vorteilhafte Ergebnisse, z.B. höhere Ausbeuten, erzielt.

Die Verbindung der Formel IIc ist neu und bildet, ebenso wie das Verfahren zu ihrer Herstellung sowie ihre Verwendung, beispielsweise als Acylierungsmittel zur Einführung von Cyclopentyloxycarbonyl, ebenfalls einen Gegenstand der vorliegenden Erfindung.

Die Verbindungen IIa und Salze davon lassen sich beispielsweise wie folgt herstellen: Man geht z.B. von einem Ester der 5-Nitroindolverbindung der Formel IId

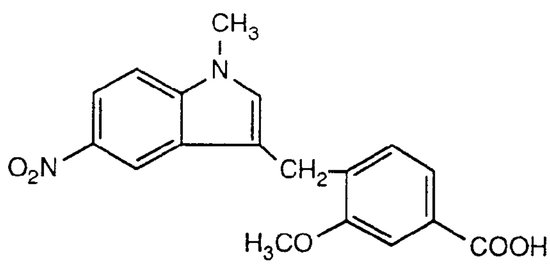

(IId)

(siehe EP 199 543) aus, hydrolysiert diesen Ester zur freien Carbonsäure der Formel IId, beispielsweise in Gegenwart einer Base oder Säure, aktiviert die Carboxygruppe und setzt die resultierende Verbindung in Analogie zu Variante c) mit einer Verbindung der Formel IVb (s. oben), oder einem Salz davon, zu einer Verbindung der Formel IIe

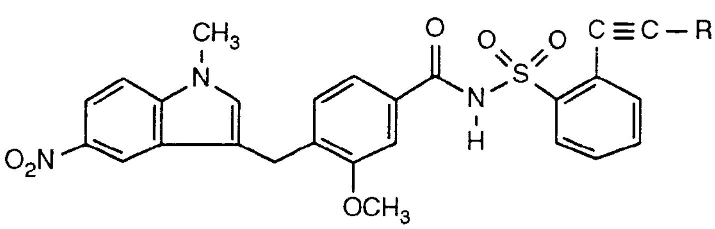

(IIe)

um. Diese wird zur entsprechenden Amino-Verbindung der Formel IIa reduziert, wobei als Reduktionsmittel

beispielsweise ein Zinn(II)halogenid, wie Zinn(II)chlorid, verwendet wird.

Die vorstehende Reaktionssequenz zur Herstellung von Verbindungen der Formel IIa bzw. I, ausgehend von Verbindungen der Formel IId über solche der Formel IIe, bildet ebenfalls einen Gegenstand der vorliegenden Erfindung.

In den Beispielen finden sich nähere Angaben zu derartigen Verfahren für die Herstellung der Ausgangsverbindungen IIa und ihrer Vorstufen.

Die Verbindungen IIb, worin X von Cyclopentyloxycarbonyloxy verschieden ist, sind bekannt oder können in Analogie zu den bekannten Verbindungen hergestellt werden.

Die Herstellung des Ausgangsmaterials der Formel IIc erfolgt durch Umsetzung von Halogenameisen-cyclopentylester, wie Chlorameisensäure-cyclopentylester, in Gegenwart eines Amins, wie Trialkylamins, z.B. N,N-Dimethyl-N-octadecylamin, unter Verwendung einer anorganischen Base, wie Alkalimetallhydroxid, z.B. Natriumhydroxid. Dabei wird vorzugsweise in einem inerten, d.h. an der Reaktion nicht beteiligten, Lösungsmittels gearbeitet.

Variante b): $X_1$ bedeutet in erster Linie Halogen, vorzugsweise Jod.

Entsprechende Verbindungen der Formel III werden mit einer Verbindung der Formel H-C≡C-R (IIIa) umgesetzt, wobei vorteilhaft unter Zusatz eines Cu-(I)-Halogenids, insbesondere Cu(I)iodid, in Gegenwart eines katalytischen Mittels, wie eines Bis-(triphenyl-phosphin)-Palladium(II)-dihalogenids, wie -dichlorids, gearbeitet wird. Insbesondere setzt man zusätzlich eine der vor- bzw. nachstehend genannten Basen, z.B. Triethylamin, zu.

Der Silylrest Z bedeutet beispielsweise Triniederalkylsilyl, wie Trimethylsilyl.

Steht $X_1$ für die Gruppe der Formel -C≡C-Z, werden entsprechende Verbindungen der Formel III beispielsweise mit einer anorganischen Base, wie einem Alkalimetall, z.B. Natriumhydroxid, behandelt und das Reaktionsgemisch anschliessend mit Hilfe einer Säure, wie Salzsäure, hydrolysiert. Dabei werden Verbindungen der Formel I erhalten, worin R Wasserstoff bedeutet.

Die Verbindungen III und deren Salze lassen sich z.B. dadurch erhalten, dass man eine Verbindung der Formel IId (s. oben) in an sich bekannter Weise [vergl. Variante c)] in eine Verbindung der Formel IIIc,

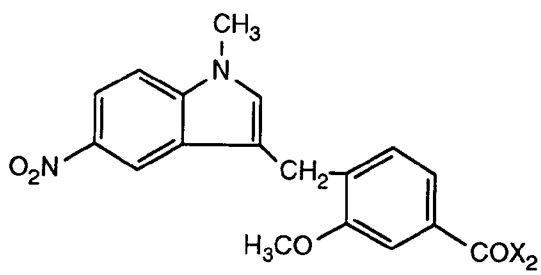

(IIIc)

worin $X_2$ für die Gruppe (IVa'), die Gruppe (IVa") oder die Gruppe (IVa"') steht, überführt und diese in Analogie zu Variante c) mit einer Verbindung der Formel IIId

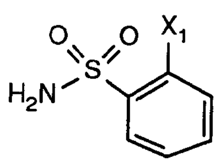

(IIId)

zu einer Verbindung der Formel IIf

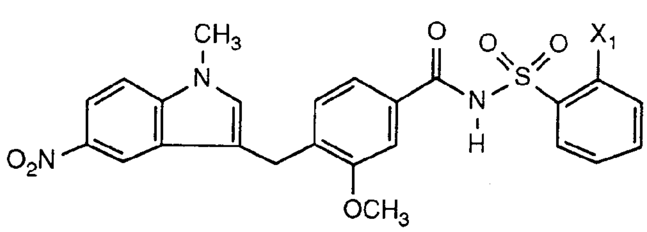

(IIf)

umsetzt. Im nächsten Reaktionsschritt erfolgt die Reduktion der Nitrogruppe auf eine dem Fachmann geläufige Weise, z.B. durch katalytische Hydrierung in Gegenwart eines Hydrierungskatalysators, wie Palladium-Kohle oder Raney-Nickel, zur Aminogruppe. Dies führt zu einer Verbindung der Formel IIIe,

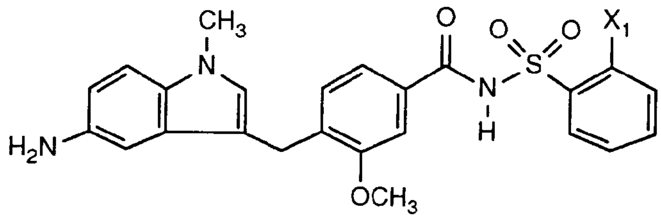
(IIIe)

die anschliessend in Analogie zu Variante a) mit einer Verbindung der Formel IIb (s. oben), insbesondere einer Verbindung der Formel IIc (s. oben), umgesetzt wird.

Alternativ kann man zur Herstellung von Verbindungen der Formel III z.B. auch zunächst in einer Verbindung der Formel IIIc, worin $X_2$ für die Gruppe (IVa'), die Gruppe (IVa'') oder die Gruppe (IVa''') steht, die Nitrogruppe auf eine dem Fachmann geläufige Weise, z.B. durch katalytische Hydrierung in Gegenwart eines Hydrierungskatalysators, wie Palladium-Kohle oder Raney-Nickel, zur Aminogruppe reduzieren und das so erhältliche Carboxy-Derivat der Formel IIIf

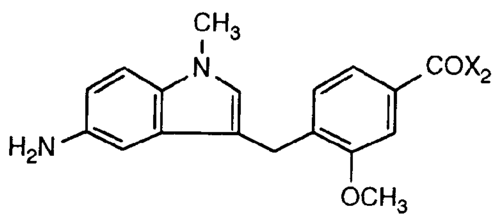
(IIIf)

mit einer Verbindung der Formel IIb (s. oben), insbesondere einer Verbindung der Formel IIc (s. oben), in Analogie zu Variante a) umsetzen. Die daraus resultierende Verbindung der Formel IVa (s. oben) wird in Analogie zu Variante c) mit einer Verbindung der Formel IIId, oder einem Salz davon, umgesetzt.

Die Aktivierung der Carboxygruppe in Verbindungen der Formel IId - zur Herstellung von Verbindungen der Formel IIIc - erfolgt vorzugsweise in Gegenwart eines Kondensationsmittels z.B. durch Umsetzung mit einer entsprechenden Hydroxyverbindung, z.B.

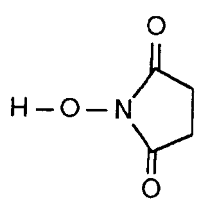
(IVd),

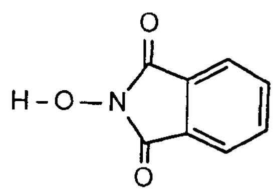
(IVe)

oder

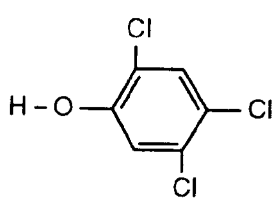
(IVf).

Als Kondensationsmittel kommt beispielsweise ein Carbodiimid, wie Diethyl- oder Dicyclohexyl-carbodiimid, oder, sofern man mit 2,4,5-Trichlorphenol umsetzt, zusätzlich eine Base, wie Diniederalkylamino-pyridin, z.B. Dimethylamino-pyridin, in Frage.

Die Umsetzung wird vorzugsweise in einem Ether, wie Tetrahydrofuran oder Dioxan, halogenierte Kohlenwasserstoffe, wie Chloroform oder Tetrachlorkohlenstoff, ferner Ester, wie Ethylacetat, oder Amide, wie Dimethylformamid, durchgeführt.

In einer vorteilhaften Variante kann man die Reduktion einer Verbindung der Formel IIIc und die Reaktion mit einer Verbindung der Formel IIc in einem Eintopfverfahren, d.h. ohne Isolierung des Zwischenprodukts der Formel IIIf, durchführen.

In den Beispielen finden sich nähere Angaben zu derartigen Verfahren für die Herstellung der Ausgangsverbindungen III und ihrer Vorstufen.

Die vorstehenden Reaktionssequenzen zur Herstellung von Verbindungen der Formel III bzw. I, ausgehend von Verbindungen der Formel IId über solche der Formeln IIIc, IIf und IIIe, oder alternativ ausgehend von einer Verbindung der Formel IIIc über solche der Formel IIIf und/oder IVa, bilden ebenfalls einen Gegenstand der vorliegenden Erfindung.

Das Ausgangsmaterial der Formel IIIa ist bekannt bzw. kann in an sich bekannter Weise hergestellt werden. Näheres kann den Ausführungsbeispielen entnommen werden.

Variante c):

Die Umsetzung gemäss Variante c) erfolgt vorzugsweise in Gegenwart einer der vorstehend genannten Basen, insbesondere sei DBU genannt.

Die Umsetzung wird vorzugsweise in einem Ether, wie Tetrahydrofuran oder Dioxan, halogenierte Kohlenwasserstoffe, wie Chloroform oder Tetrachlorkohlenstoff, ferner Ester, wie Ethylacetat, oder Amide, wie Dimethylformamid, durchgeführt.

Die Verbindungen IVa und deren Salze lassen sich beispielsweise wie folgt herstellen.

Man geht von einer Verbindung der Formel IIIc (s. oben) aus und reduziert darin die Nitrogruppe auf eine dem Fachmann geläufige Weise, z.B. durch katalytische Hydrierung in Gegenwart eines Hydrierungskatalysators, wie Palladium- Kohle oder Raney-Nickel, zur Aminogruppe. Im nächsten Reaktionsschritt wird die so erhältliche Verbindung der Formel IIIf (s. oben) mit einer Verbindung der Formel IIb, insbesondere einer Verbindung der Formel IIc, in Analogie zu Variante a) in die betreffende Verbindung der Formel IVa überführt.

Die Reduktion der 5-Nitroindolverbindung sowie die Umsetzung mit einer Verbindung der Formel IIc kann vorteilhaft in einem Eintopfverfahren, d.h. ohne Isolierung einer Verbindung der Formel IIIf, durchgeführt werden.

Die Erfindung betrifft ebenfalls die nach den vorstehenden Verfahrensvarianten erhältlichen neuen Verbindungen.

Eine erfindungsgemäss oder auf andere Weise erhältliche Verbindung der Formel I oder Salz davon kann in an sich bekannter Weise in eine andere Verbindung der Formel I übergeführt werden.

Verbindungen der Formel I, worin $R_1$ Hydroxy ist, können in an sich bekannter Weise zu solchen Verbindungen der Formel I acyliert werden, worin $R_1$ $C_2$-$C_7$-Alkanoyloxy ist, beispielsweise analog der in Variante c) geschilderten Methodik, z.B. durch Umsetzung mit der entsprechenden Carbonsäure oder einem reaktionsfähigen Derivat davon. Derartige reaktionsfähige Derivate sind beispielsweise Anhydride, inklusive gemischte Anhydride, wie ein Säurehalogenid, z.B. -chlorid, oder Anhydride mit einem Ameisensäureester, aktivierte Carbonsäureester, wie Cyanmethyl-, (4-)Nitrophenyl-, Polyhalogenphenyl-, z.B. Trichlorphenyl-, -ester. Die Umsetzung mit der Carbonsäure oder einem Salz davon erfolgt unter wasserabspaltenden Bedingungen, z.B. unter azeotroper Entfernung des Reaktionswassers, oder durch Behandeln mit einem geeigneten Kondensationsmittel, z.B. N,N′-Dicyclohexyl-carbodiimid. Die Umsetzung mit einem reaktionsfähigen Säurederivat wird vorteilhaft in Gegenwart einer Base durchgeführt.

Durch Behandeln mit starken Basen, wie Alkalimetallhydroxiden, z.B. Lithiumhydroxid, kann $R_1$ = $C_2$-$C_7$-Alkanoyloxy zu $R_1$ = OH hydrolysiert werden.

Bedeutet $R_1$ Hydroxy, kann dieses in an sich bekannter Weise zu solchen Verbindungen der Formel I verethert werden, worin $R_1$ $C_1$-$C_7$-Alkoxy oder Phenyl-$C_1$-$C_7$-alkoxy bedeutet. Die Veretherung kann z.B. mit einem Alkohol, wie einem gegebenenfalls substituierten Niederalkanol, oder einem reaktionsfähigen Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide oder Methan-, Benzol- oder p-Toluol-sulfonate, in Betracht. Die Veretherung kann z.B. in Gegenwart einer Base, z. B. eines Alkalimetall-hydrids, -hydroxids oder -carbonats oder eines basischen Amins, erfolgen. Umgekehrt können entsprechende Ether, wie Niederalkoxyverbindungen, z.B. mittels starker Säuren, wie Mineralsäuren, z.B. Brom- oder Iod-wasserstoffsäure, die vorteilhaft in Form von Pyridiniumhalogeniden vorliegen können, oder mittels Lewissäuren, z.B. Halogeniden von Elementen der III. Hauptgruppe oder der entsprechenden Nebengruppen, gespalten werden. Diese Umsetzungen können, falls erforderlich, unter Kühlen oder Erwärmen, z.B.

in einem Temperaturbereich von etwa -20° bis etwa +100°C, in An- oder Abwesenheit eines Lösungs- oder Verdünnungsmittels, unter Inertgas und/oder unter Druck und gegebenenfalls in einem geschlossenen Gefäss durchgeführt werden.

Entsprechende Aether der Formel I, worin $R_1$ (Phenyl-)$C_1$-$C_7$-Alkoxy bedeutet, können in an sich bekannter Weise gespalten werden, beispielsweise mittels starker Säuren, wie Mineralsäuren, z.B. Brom- oder Iod-wasserstoffsäure, die vorteilhaft in Form von Pyridiniumhalogeniden vorliegen können, oder mittels Lewissäuren, z.B. Halogeniden von Elementen der III. Hauptgruppe oder der entsprechenden Nebengruppen, gespalten werden. Diese Umsetzungen können, falls erforderlich, unter Kühlen oder Erwärmen, z.B. in einem Temperaturbereich von etwa -20° bis etwa +100°C, in An- oder Abwesenheit eines Lösungs- oder Verdünnungsmittels, unter Inertgas und/oder unter Druck und gegebenenfalls in einem geschlossenen Gefäss durchgeführt werden.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens und Salze mit Basen durch Behandeln mit einer geeigneten Base oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z.B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Infolge der engen Beziehung zwischen der Verbindung I in freier Form und in Form ihrer Salze sind im vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Verbindungen I und ihre Salze können, je nach Wahl der Ausgangsstoffe und Arbeitsweisen, in Form eines der möglichen Isomeren oder als Gemisch derselben vorliegen. Dabei sind als reine Isomere z.B. reine Diastereomere erhältlich. Entsprechend können als Isomerengemische z.B. Diastereomerengemische vorliegen. Verfahrensgemäss oder anderweitig erhältliche Isomerengemische von Verbindungen I in freier Form oder in Salzform können in üblicher Weise in die Komponenten aufgetrennt werden, z. B. aufgrund der physikalisch-chemischen Unterschiede der Bestandteile in bekannter Weise durch fraktionierte Kristallisation, Destillation und/oder Chromatographie. Vorteilhaft isoliert man das wirksamere Isomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. .Salzes verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen. Neue Ausgangsstoffe und Zwischenprodukte, jeweils in freier Form oder in Salzform, für die Herstellung der Verbindungen I bzw. ihrer Salze, ihre Verwendung und Verfahren zu ihrer Herstellung bilden ebenfalls einen Gegenstand der Erfindung, wobei die Variable R die für die Verbindungen I angegebene Bedeutung hat.

Die Erfindung betrifft ebenfalls die Verwendung der Verbindungen I und ihrer pharmazeutisch verwendbaren Salze zur Behandlung von allergischen Zuständen und Erkrankungen,vorzugsweise in Form von pharmazeutisch verwendbaren Zubereitungen, insbesondere in einem Verfahren zur therapeutischen Behandlung des tierischen oder menschlichen Körpers, sowie ein solches Behandlungsverfahren.

Die Erfindung betrifft gleichfalls pharmazeutische Präparate, die eine Verbindung I oder ein pharmazeutisch verwendbares Salz davon als Wirkstoff enthalten, sowie Verfahren zu ihrer Herstellung. Bei diesen pharmazeutischen Präparaten handelt es sich um solche zur enteralen, wie oralen, ferner rektalen, Verabreichung, um solche zur parenteralen Verabreichung, um solche zur lokalen Verabreichung und vor allem um solche zur Inhalationsverabreichung an Warmblüter, vor allem an Menschen, wobei der pharmakologische Wirkstoff allein oder zusammen mit üblichen pharmazeutischen Hilfsstoffen enthalten ist.

Die pharmazeutischen Präparate enthalten (in Gewichtsprozenten) z.B. von etwa 0,001 % bis 100 %, vorzugsweise von etwa 0,1 % bis etwa 50 %, des Wirkstoffs.

Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, herpestellt. So kann man pharmazeutische Präparate zur oralen Anwendung erhalten, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten, Ueberzügen versehen, wobei man u.a. konzentrierte Zuckerlösungen, welche gegebenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Pharmazeutische Präparate zur lokalen Verabreichung sind z. B. für die topische Behandlung der Haut Lotionen, Crèmes und Salben, d. h. flüssige oder semifeste Oel-in-Wasser- oder Wasser-in-Oel-Emulsionen, Fettsalben, die wasserfrei sind, Pasten, d. h. Crèmes und Salben mit sekretabsorbierenden Puderbestandteilen, Gele, die wässrig, wasserarm oder wasserfrei sind und aus quellbaren, gelbildenden Materialien bestehen, Schäume, d. h. in Aerosolform vorliegende flüssige Oel-in-Wasser-Emulsionen, welche aus Druckbehältern verabreicht werden, und eine wässrig-ethanolische Grundlage aufweisende Tinkturen, welche jeweils weitere übliche pharmazeutische Hilfsstoffe, wie Konservierungsmittel, enthalten können. Für die lokale Behandlung der Augen eignen sich z. B. Augentropfen, welche den Wirkstoff in steriler wässriger oder öliger Lösung enthalten, und Augensalben, die vorzugsweise ebenfalls in steriler Form hergestellt werden. Für die lokale Behandlung der Nase sind z. B. Sprays, ähnlich den weiter unten beschriebenen für die Behandlung der Atemwege, grobe Puder, die durch schnelles Inhalieren durch die Nasenlöcher verabreicht werden, und vor allem Nasentropfen, welche den Wirkstoff in wässriger oder öliger Lösung enthalten, geeignet. Für die lokale Behandlung der Mundhöhle eignen sich z. B. Lutschbonbons und Pastillen, welche den Wirkstoff in einer z. B. aus Zucker und Gummiarabikum oder Tragantgummi gebildeten inerten Masse enthalten, welcher Geschmacksstoffe beigegeben sein können. Die Herstellung der pharmazeutischen Präparate zur lokalen Verabreichung erfolgt in an sich bekannter Weise durch Vermischung des Wirkstofts mit den pharmazeutischen

Hilfsstoffen, z. B. durch Lösen oder Suspendieren des Wirkstoffs in der Grundlage oder in einem Teil davon, falls notwendig. Zur Herstellung von Emulsionen, in denen der Wirkstoff in einer der flüssigen Phasen gelöst ist, wird der Wirkstoff in der Regel vor der Emulgierung in dieser gelöst; zur Herstellung von Suspensionen, in denen der Wirkstoff in der Emulsion suspendiert ist, wird der Wirkstoft nach der Emulgierung mit einem Teil der Grundlage vermischt und dann dem Rest der Formulierung beigegeben.

Pharmazeutische Präparate zur Inhalationsverabreichung sind solche, in denen der Wirkstoff in mikronisierter Form vorliegt, d. h. in denen die Partikelgrösse des Wirkstoffs weniger als 20 µm, insbesondere weniger als 10 µm und vorteilhaft weniger als 5 µm, beträgt, z. B. mikronisierte Pulver und Aerosole, die in Form von Sprays verabreicht werden. Die mikronisierten Pulver enthalten den Wirkstoff allein oder zusammen mit einem inerten Trägerstoff, wie Lactose, vorteilhaft zusammen mit einem der nachstehend erwähnten Treibmittel. Aerosole sind Lösungen, Suspensionen oder Emulsionen des Wirkstoffs in einer geeigneten, pharmazeutisch verwendbaren, flüssigen Phase, wie in Ethanol oder Wasser oder einem entsprechenden Gemisch, können nach Bedarf auch andere pharmazeutische Hilfsstoffe, wie nicht-ionische oder anionische oberflächenaktive Mittel, Emulgatoren und Stabilisatoren, und/oder Wirkstoffe anderer Art aufweisen und enthalten ein Treibmittel, z. B. ein inertes Gas, wie Butan, unter erhöhtem Druck oder insbesondere eine leicht flüchtige, vorzugsweise unter normalem Druck unterhalb der üblichen Raumtemperatur (z.B. zwischen etwa -30°C und etwa +10°C) siedende, Flüssigkeit, wie ein mindestens teilweise fluoriertes polyhalogeniertes Niederalkan, oder ein Gemisch solcher Flüssigkeiten. Zur Herstellung der pharmazeutischen Präparate in zur Inhalationsverabreichung fertiger Form wird eine entsprechende pharmazeutische Zusammensetzung zusammen mit dem Treibmittel in geeignete Behälter, wie Flacons oder Druckflaschen, abgefüllt, welche mit einer geeigneten Versprühungseinrichtung, z. B. einem Ventil, versehen sind. Das Ventil ist vorzugsweise als Dosierungsventil konstruiert, welches bei Betätigung eine vorbestimmte Menge des Behälterinhalts, entsprechend einer vorbestimmten Dosis des Wirkstoffs, freigibt. Bei der Herstellung der fertigen Arzneimittelform kann man auch so vorgehen, dass entsprechende Mengen der pharmazeutischen Zusammensetzung und des Treibmittels separat in die Behälter abgefüllt werden und erst dort zur Vermischung kommen.

Die Dosierung des Wirkstoffs kann von verschiedenen Faktoren, wie Wirksamkeit und Wirkungsdauer des Wirkstoffs, Stärke der zu behandelnden Krankheit bzw. ihrer Symptome, Applikationsweise, Warmblüter-Spezies, -Geschlecht, -Alter, -Gewicht und/oder individuellem Zustand des Warmblüters, abhängen. Im Normalfall ist für einen etwa 75 kg schweren Warmblüter eine ungefähre Tagesdosis von etwa 10 mg bis etwa 1500 mg, insbesondere von etwa 25 bis etwa 250 mg, gegebenenfalls in mehreren, gegebenenfalls gleichen, Teildosen, zu veranschlagen.

Die nachfolgenden Beispiele illustrieren die vorstehend beschriebene Erfindung, ohne sie in ihrem Umfang in irgendeiner Weise einzuschränken. Temperaturen sind in Celsiusgraden angegeben. "DMSO" steht für "Dimethylsulfoxid".

Beispiel 1:

N-[4-[5-(Cyclopentyloxycarbonyl-amino)-1-methyl-indol-3-yl-methyl]-3-methoxy-benzoyl]-2-(5-methoxy-pent-1-in-1-yl)-benzolsulfonamid

Zu einer Lösung von 1,3 g 4-[5-(Cyclopentyloxycarbonyl-amino)-1-methyl-indol-3-yl-methyl]-3-methoxy-benzoesäure-N-succinimidester und 0,7 g 2-(5-Methoxy-pent-1-in-1-yl)-benzolsulfonamid in 25 ml Methylenchlorid werden 1,15 ml 1,8-Diazabicyclo-[5.4.0] undec-7-en (DBU) zugegeben. Die Mischung wird drei Stunden unter Argon gerührt und dann mit 100 ml Methylenchlorid verdünnt. Die Methylenchlorid-Phase wird zweimal mit je 50 ml 1n Salzsäure, 50 ml Wasser und 50 ml gesättigter Kochsalzlösung gewaschen, über Magnesiumsulfat getrocknet und bei 20 Torr und 40° eingedampft. Der Rückstand wird auf 100 g Kieselgel (Merck, Si 60, 40-63 µm) chromatographiert, wobei mit Ethylacetat eluiert wird. Die Fraktionen, welche die Titelverbindung enthalten, werden bei 20 Torr und 40° eingedampft. Auf diese Weise wird ein weisser amorpher Feststoff erhalten. $^1$H-NMR (400 MHz, DMSO-$d_6$): 8.88 (s, b, 1H); 7.95 (m, 1H); 7.59 (d, 1H); 7.58 (d, 1H); 7.45 (d, b, 1H); 7.34 (m, b, 3H); 7.23 (d, 1H); 7.17 (d, d, 1H); 7.02 (d, 1H); 6.91 (s, 1H); 5.09 (m, 1H); 3.93 (s, 2H); 3.86 (s, 3H); 3.67 (s, 3H); 3.39 (t, 2H); 3.18 (s, 3H); 2.35 (t, 2H); 1.92-1.55 (m, 8H); 1.67 (m, 2H).

Das Ausgangsmaterial wird wie folgt hergestellt:

a) 10,0 g 3-Methoxy-4-(1-methyl-5-nitro-indol-3-yl-methyl)-benzoesäuremethylester (vergl. EP 199543 oder J. Med. Chem. 1990, 33, 1781) werden in 240 ml Tetrahydrofuran und 180 ml Methanol gelöst. Die Lösung wird mit einer Lösung von 4,2 g Lithiumhydroxid-monohydrat in 70 ml Wasser versetzt. Die Mischung wird 15 Stunden bei Raumtemperatur gerührt und unter 11 Torr bei 50° auf ein Volumen von ca. 50 ml eingeengt. Man verdünnt mit 50 ml Wasser und stellt die Lösung mit 1-n Salzsäure sauer. Die ausgeschiedenen gelben Kristalle werden abfiltriert, mit 20 ml Wasser gewaschen, und unter 0.01 Torr bei 40° während 20 Stunden getrocknet. Die 4-(1-Methyl-5-nitro-indol-3-yl-methyl)-3-methoxy-benzoesäure

schmilzt bei 263-265°.

b) Zu einer Suspension von 3,4 g 4-(1-Methyl-5-nitro-indol-3-yl-methyl)-3-methoxybenzoesäure in 100 ml Tetrahydrofuran werden 1,23 g N-Hydroxysuccinimmid und 2,38 g Dicyclohexylcarbodiimid gegeben. Diese Mischung wird während 16 Std. bei Raumtemperatur unter Argon gerührt. Anschliessend wird das Reaktionsgemisch filtriert und das Filtrat bei 15 Torr und 40° eingedampft, der Rückstand wird in heissem Ethylacetat gelöst, beim Abkühlen kristallisiert 3-Methoxy-4-(1-methyl-5-nitro-indol-3-yl-methyl)-benzoesäure-N-succinimidester in Form gelber Kristalle aus, Schmelzpunkt 201-203°.

Auf alternativem Wege kann man den 3-Methoxy-4-(1-methyl-5-nitro-indol-3-yl-methyl)-benzoesäure-N-succinimidester wie folgt herstellen:

36,0 g 3-Methoxy-4-methylbenzoesäuremethylester werden in 300 ml Methanol gelöst. Der Lösung setzt man tropfenweise 25 ml 30%ige Natronlauge zu. Die Mischung wird 16 Stunden bei Raumtemperatur und 3 Stunden bei 40° gerührt und unter 11 Torr bei 50° eingedampft. Den Rückstand löst man in 500 ml Wasser und stellt die Lösung mit conc. Salzsäure sauer (pH=1). Die ausgeschiedenen weissen Kristalle werden abfiltriert, mit 40 ml Wasser gewaschen und 15 Stunden unter 0,1 Torr bei 90° getrocknet. Die 3-Methoxy-4-methylbenzoesäure schmilzt bei 157-158°.

Zu einer Lösung von 3,93 g 3-Methoxy-4-methylbenzoesäure in 100 ml Tetrahydrofuran werden unter Rühren unter Argon 2,9 g N-Hydroxysuccinimid und 5,6 g Dicyclohexylcarbodiimid zugegeben. Das Gemisch wird 18 stunden bei Raumtemperatur gerührt. Die weisse Suspension wird abfiltriert und mit 40 ml Tetrahydrofuran nachgewaschen, Das Filtrat wird unter 11 Torr bei 40° zur Trockene eingedampft. Den Rückstand löst man in 30 ml heissem Ethylacetat. Nach Abkühlen der Lösung und Zusatz von 10 ml Ether kristallisiert der 3-Methoxy-4-methylbenzoesäure-N-succinimidester in Form weisser Kristalle vom Smp. 134-135°.

Eine Suspension von 14,05 g 3-Methoxy-4-methylbenzoesäure-N-succinimidester in 160 ml Tetrahydrofuran wird unter Rühren bei Raumtemperatur unter Stickstoff mit 10,95 g N-Bromsuccinimid und 0,5 g Azoisobutyronitril versetzt. Die Mischung wird 24 Stunden unter Rückfluss erhitzt, abgekühlt auf Raumtemperatur und abfiltriert. Das Filtrat wird unter 11 Torr bei 50° eingedampft. Den Rückstand Kristallisiert man aus einem Gemisch von 100 ml Ethylacetat und 200 ml Hexan. Der 4-Brommethyl-3-methoxy-benzoesäure-N-succinimidester schmilzt bei 124-125°.

2,85 g N-Methyl-5-nitroindol und 7,5 g Silbercarbonat werden unter Rühren in einer Argonatmosphäre in 100 ml Toluol suspendiert. Die Suspension wird 18 Stunden unter Rückfluss erhitzt, auf 55° abgekühlt und unter Rühren tropfenweise mit einer Lösung von 5,4 g 4-Brommethyl-3-methoxy-benzoesäure-N-succinimidester versetzt. Die Mischung wird fünf Tage bei 55-60° gerührt, auf Raumtemperatur abgekühlt und abfiltriert. Der Rückstand wird mit 30 ml Toluol nachgewaschen. Das Filtrat wird unter 11 Torr bei 50° eingedampft. Der Rückstand wird an 1000 g Silicagel über eine MPLC-Kolonne chromatographiert (Elutionsmittel: Ethylacetat/Hexan 3:2). Der auf diese Weise erhältliche 3-Methoxy-4-(1-methyl-5-nitro-indol-3-yl-methyl)-benzoesäure-N-succinimidester wird aus Ethylacetat umkristallisiert. Man erhält gelbe Kristalle vom Smp. 215-216°.

c) 3,0 g 3-Methoxy-4-(1-methyl-5-nitro-indol-3-yl-methyl)-benzoesäure-N-succinimidester werden in 60 ml Tetrahydrofuran gelöst und 0,6 g 5 % Palladium auf Aktivkohle zugegeben. Während 10 Stunden wird unter Normaldruck bei Raumtemperatur hydriert. Dann wird der Katalysator mit Hilfe einer Glasfritte abgesaugt und mit heissem Tetrahydrofuran nachgewaschen. Das Filtrat wird bei 15 Torr und 40° eingedampft. Der entstandene Feststoff wird mit Ethylether aufgerührt. Nach dem Filieren erhält man 3-Methoxy-4-(1-methyl-5-amino-indol-3-yl-methyl)-benzoesäure-N-succinimidester in Form beiger Kristalle. $^1$H-NMR (200 MHz, DMSO-$d_6$): 7.61 (d, d, 1H); 7.55 (s, 1H); 7.23 (d, 1H); 7.09 (d, 1H); 6.92 (s, 1H); 6.50-6.60 (m, 2H); 4.50 (s, b, 2H); 3.96 (2S, 5H); 3.63 (s, 3H); 2.90 (s, 4H).

d) Zu einer Lösung von 2,4 g 3-Methoxy-4-(1-methyl-5-amino-indol-3-yl-methyl)-benzoesäure-N-succinimidester in 70 ml Methylenchlorid werden 1,8 g N-Methylmorpholin und 0,95 g Chlorameisensäurecyclopentylester gegeben. Diese Reaktionsmischung wird während 2 Std. bei Raumtemperatur und unter Argon gerührt. Anschliessend wird das Reaktionsgemisch mit 20 ml eiskalter 1n Salzsäure versetzt. Die organische Phase wird abgetrennt und mit 20 ml 0,1n Salzsäure, mit 30 ml Wasser und mit 20 ml gesättigter Kochsalzlösung gewaschen, getrocknet ($MgSO_4$) und bei 15 Torr und 40° eingedampft. Zurück bleibt ein hellrotes Oel, welches aus heissem Ethylacetat kristallisiert. Der 4-[5-(Cyclopentyloxycarbonyl-amino)-1-methyl-indol-3-yl-methyl]-3-methoxy-benzoesäure-N-succinimidest er schmilzt bei 195-196°.

e) 2,4 g Natriumhydrid-Dispersion in Oel werden zweimal mit 20 ml Hexan aufgeschlämmt. Das Hexan wurde jeweils abdekantiert. Anschliessend werden 100 ml Tetrahydrofuran und dann innert 5 Minuten 4,62 ml Pent-4-in-1-ol, welches in 30 ml Tetrahydrofuran gelöst ist, zugegeben. Die Suspension wird auf 40° erhitzt und 30 Minuten gerührt, worauf eine Lösung von 3,42 ml Methyljodid in 20 ml Tetrahydrofuran zugetropft wird. Das Reaktionsgemisch wird unter Argon für 16 Stunden bei Raumtemperatur gerührt. Das Tetrahydrofuran wird bei Normaldruck abdestilliert, der Rückstand in 100 ml Aether/Wasser = 1/1 aufgenommen, und mit 1n Salzsäure auf pH = 2 gebracht. Im Scheidetrichter wird die organische Phase abgetrennt und die wässrige Phase noch zweimal mit je 20 ml Aether extrahiert. Die vereinigten organischen Phasen werden mit Wasser gewaschen, über Magnesiumsulfat getrocknet und das Lösungsmittel wird bei Normaldruck abgedampft. Zurück bleibt Pent-4-in-1-yl-methylether als gelbes Oel. Das $^1$H-NMR zeigt einen Gehalt von 60 % Pent-4-in-1-yl-methylether in Ethylether/Tetrahydrofuran = 1/1 an. $^1$H-NMR (200 MHz, DMSO-$d_6$): 3,37 (t, 2H); 3,23 (s, 3H); 2,77 (t, 1H); 2,19 (d, t, 2H); 1,68 (m, 2H).

Analog können hergestellt werden:

But-3-in-1-yl-methylether als 20 % Lösung in Tetrahydrofuran/Ethylether = 3/1. $^1$H-NMR (200 MHz, $CDCl_3$): 3,45 (m, 2H); 3,35 (s, 3H); 2,42 (m, 2H); 1,96 (t, 1H); ausgehend von But-3-in-1-ol.

Prop-2-in-1-yl-methylether als 10 % Lösung in Tetrahydrofuran/Ethylether = 1/2. $^1$H-NMR (200 MHz, $CDCl_3$): 4,07 (m, 2H); 3,35 (s, 3H); 2,41 (t, 1H); ausgehend von Prop-2-in-1-ol.

f) 135 ml N,N-Dimethylformamid und 22 ml Triethylamin werden vorgelegt und 7.5 g 2-Jod-benzolsulfonamid, 2,6 g Pent-4-in-1-yl-methylether sowie 0,067 g Kupfer-(I)-iodid und 0,144 g Bis(triphenylphosphin)palladium-(II)-dichlorid zugegeben. Das Reaktionsgemisch wird auf 65° erwärmt und 22 Stunden bei dieser Temperatur unter Argon gerührt. Nach dieser Zeit wird das N,N-Dimethylformamid bei 0,1 Torr und 40° abdestilliert. Das Rohprodukt wird auf 500 g Kieselgel (Merck Si 60, 40-63 µm) mit Hexan/Ethylacetat = 2/1 als Laufmittel gereinigt. Die das Produkt enthaldende Fraktionen werden bei 15 Torr und 40° eingedampft. Auf diese Weise erhält man weisses kristallines 2-(5-Methoxy-pent-1-in-1-yl)-benzolsulfonamid, Schmelzpunkt: 59-61 °.

Analog können hergestellt werden:

2-(Pent-1-in-1-yl)-benzolsulfonamid, hellbraune Kristalle. $^1$H-NMR (200 MHz), DMSO-$d_6$): 7.90 (d, d, 1H); 7.45-7.61 (m, 3H); 7.18 (s, 2H); 2.49 (t, 2H); 2.60 (m, 2H); 1.02 (t, 3H); ausgehend von 2-Jod-benzolsulfonamid und 1-Pentin.

2-(3-Methyl-but-1-in-1-yl)-benzolsulfonamid, gelbliches Oel, das sich beim Stehenlassen verfestigt. $^1$H-NMR (200 MHz), DMSO-$d_6$): 7.90 (d, d, 1H); 7.45-7.60 (m, 3H); 7.12 (s, 2H); 2.88 (m, 1H); 1.25 (s, 3H); 1.20 (s, 3H); ausgehend von 2-Jod-benzolsulfonamid und 3-Methyl-1-butin.

2-(3-Methoxy-prop-1-in-1-yl)-benzolsulfonamid, gelbliches Oel, welches sich beim Stehenlassen verfestigt. $^1$H-NMR (200 MHz), $CDCl_3$): 8.02 (d, d, 1H); 7.59 (d, t, 1H); 7.40-7.55 (m, 2H); 5.30 (s, 2H); 4.38 (s, 2H); 3.48 (s, 3H); ausgehend von 2-Jod-benzolsulfonamid und 3-Methoxy-prop-1-in.

2-(4-Methoxy-but-1-in-1-yl)-benzolsulfonamid, hellbraune Kristalle. $^1$H-NMR (200 MHz), DMSO-$d_6$): 7.91 (d, d, 1H); 7.47-7.62 (m, 3H); 7.20 (s, 2H); 3.57 (t, 2H); 3.31 (s, 3H); 2.77 (t, 2H); ausgehend von 2-Jod-benzolsulfonamid und 4-Methoxy-butin.

2-(5-Hydroxy-pent-1-in-1-yl)-benzolsulfonamid, rötliches Oel. $^1$H-NMR (300 MHz), DMSO-$d_6$): 7.88 (d, d, 1H); 7.45-7.60 (m, 3H); 7.20 (s, 2H); 4.59 (t, 1H); 3.55 (q, 2H); 2.53 (t, 2H); 1.71 (m, 2H); ausgehend von 2-Jod-benzolsulfonamid und 5-Hydroxy-pentin.

2-(4-Hydroxy-but-1-in-1-yl)-benzolsulfonamid, hellbraune Kristalle. $^1$H-NMR (400 MHz), DMSO-$d_6$): 7.88 (d, d, 1H); 7.45-7.60 (m, 3H); 7.30 (s, b, 2H); 5.30 (s, b, 1H); 3.62 (6, 2H); 2.62 (t, 2H); ausgehend von 2-Jod-benzolsulfonamid und 4-Hydroxy-butin.

2-(Hydroxy-prop-1-in-1-yl)-benzolsulfonamid, weisse Kristalle. $^1$H-NMR (400 MHz), $CDCl_3$): 8.20 (d, d, 1H); 7.60 (d, d, 1H); 7.52 (d, t, 2H); 7.46 (d, t, 1H); 5.58 (s, 2H); 4.60 (s, 2H); ausgehend von 2-Jod-benzolsulfonamid und 3-Hydroxy-propin.

2-(3-Hydroxy-but-1-in-1-yl)-benzolsulfonamid, orange Kristalle. $^1$H-NMR (400 MHz), $CDCl_3$): 8.03 (d, d, 1H); 7.60 (d, d, 1H); 7.55 (d, t, 1H); 7.47 (d, t, 1H); 5.40 (s, 2H); 4.86 (q, 1H); 3.05 (s, b, 1H); 1.61 (d, 3H); ausgehend von 2-Jod-benzolsulfonamid und 3-Hydroxy-butin.

2-(4-Hydroxy-pent-1-in-1-yl)-benzolsulfonamid, gelbes Oel. $^1$H-NMR (200 MHz), DMSO-$d_6$): 7.90 (d, d, 1H); 7.45-7.63 (m, 3H); 7.36 (s, 2H); 5.30 (d, 1H); 3.92 (m, 1H); 2.65 (d, d, 1H); 2.50 (d, d, 1H); 1,21 (d, 3H); ausgenhend von 2-Jod-benzolsulfonamid und 4-Hydroxypentin.

Beispiel 2:

N-[4-[5-(Cyclopentyloxycarbonyl-amino)-1-methyl-indol-3-yl-methyl]3-methoxy-benzoyl]-2-(5-methoxy-pent-1-in-1-yl)-benzolsulfonamid kann auch analog zu Beispiel 1 ausgehend von 4-[5-(Cyclopentyloxycarbonyl-amino)-1-methyl-indol-3-yl-methyl]-3-methoxy-benzoesäure-2,4,5-trichlor-phenylester (Smp. 134-136°) und 2-(5-Methoxy-pent-1-in-1-yl)-benzolsulfonamid hergestellt werden.

Das Ausgangsgmaterial wird wie folgt hergestellt:

Eine Mischung von 2,38 g 4-(1-Methyl-5-nitro-indol-3-yl-methyl)-3-methoxy-benzoesäure, 1,48 g 2,4,5-Trichlorphenol und 1,0 g 4-Dimethylaminopyridin in 140 ml Tetrahydrofuran wird unter Rühren in einer Sticksoffatmosphäre bei Raumtemperatur mit 1,66 g Dicyclohexylcarbodiimid versetzt. Das Reaktionsgemisch wird 15 Stunden bei Raumtemperatur gerührt und filtriert. Das Filtrat wird unter 11 Torr bei 50° eingedampft. Der Rückstand wird in 400 ml Chloroform unter Erwärmen gelöst. Man wäscht die organische Phase bei Raumtemperatur mit 50 ml 2-n Salzsäure und 50 ml Wasser, trocknet sie über $MgSO_4$ und engt die Lösung unter 11 Torr bei 40° zur Trockene ein. Der Rückstand wird an 350 g Silicagel chromatographiert (Eluierungsmittel: Dichlormethan). Der auf diese Weise erhältliche 3-Methoxy-4-(1-methyl-5-nitro-indol-3-yl-methyl)benzoesäure-2,4,5-trichlorphenylester wird mit 50 ml Ether aufgeschlämmt und abfiltriert. Man erhält gelbe Kristalle vom Smp. 235-236°.

Eine Suspension von 1,04 g 3-Methoxy-4-(1-methyl-5-nitro-indol-3-yl-methyl)-benzoesäure-2,4,5-trichlorphenylester, 0,6 g Dicyclopentyl-dicarbonat und 1,2-Dichlorbenzol in 60 ml Tetrahydrofuran wird nach Zusatz von 0,4 g Palladium-Kohle-Katalysator (10%) 25 Stunden bei Raumtemperatur hydriert. Es werden nochmals 0,8 g Katalysator und 0,5 g 1,2-Dichlorbenzol zugesetzt und weitere 45 Stunden hydriert. Zur Abtrennung des Katalysators wird die Mischung filtriert und der Rückstand mit 50 ml Tetrahydrofuran nachgewaschen. Das Filtrat wird unter 11 Torr zur Trockene eingedampft. Der Rückstand wird mit 100 ml Petrolether verrieben, abfiltriert und an 190 g Silicagel über eine MPLC-Kolonne chromatographiert (Eluierungsmittel: Dichlormethan). Der so erhältliche 4-[5-(Cyclopentyloxycarbonylamino)-1-methyl-indol-3-yl-methyl] benzoesäure-2,4,5-tri-chlorphenylester wird aus Ether/Petrolether umkristallisiert. Man erhält farblose Kristalle vom Smp. 134- 136°.

Herstellung von Dicyclopentyl-dicarbonat: Eine Lösung von 8,29 g Chlorameisensäure-cyclopentylerter in 64 ml Dichlormethan wird bei 20° mit 15 mg N,N-Dimethyl-octadecylamin und anschliessend unter schnellem Rühren mit einer Lösung von 2,0 g Natriumhydroxid in 30 ml Wasser versetzt. Die Mischung wird 20 Minuten bei 20° gerührt. Man trennt die organische Phase ab, trocknet sie über Calciumchlorid, filtriert ab, rührt das Filtrat 5 Minuten mit 1,0 g Hyflo Super Cel, filtriert und engt das Filtrat unter 11 Torr bei 30° zur Trocknung ein. Vom Rückstand werden die leichter flüchtigen Anteile durch Destillation bei 0,03 Torr und 80° abgetrennt. Der Destillationsrückstand, das Dicyclopentyl-dicarbonat, liegt als farblose Flüssigkeit vor. Ausbeute 80 %, Kp. 106/0,03 Torr (Zersetzung).

<u>Beispiel 3:</u>

In analoger Weise wie in Beispiel 1 beschrieben wird hergestellt: <u>N-[4-[5-(Cyclopentyloxycarbonyl-amino)-1-methyl-indol-3-yl-methyl]-3-methoxybenzoyl]-2-ethinyl-benzolsulfonamid</u>, weisser amorpher Feststoff: $^1$H-NMR (400 MHz, $CDCl_3$): 9.09 (s, b, 1H); 8.34 (m, 1H); 7.67 (m, 1H); 7.59 (m, 2H); 7.53 (b, 1H); 7.36 (d, 1H); 7.25-7.10 (m, 4H); 6.79 (s, 1H); 6.57 (b, 1H); 5.23 (m, 1H); 4.07 (s,2H); 3.88 (s, 3H); 3.73 (s, 3H); 3.64 (3.64 (s, 1H); 1.97-1.58 (m, 8H);
ausgehend von 4-[5-(Cyclopentyloxycarbonyl-amino)-1-methyl-indol-3-yl-methyl]-3-methoxy-benzoesäure-N-succinimidester und 2-Ethinyl-benzolsulfonamid.

Das Ausgangsmaterial wird wie folgt hergestellt:

a) Eine Lösung von 226,7 g 2-Nitro-benzolsulfonammid [Beilstein, Band 11 H, S.68; E I, S. 20; E III,S.115] in 2270 ml Methanol wird nach Zusatz von 23 g Raney-Nickel während 5 Stunden bei 30-40° unter Normaldruck hydriert. Zur Abtrennung des Katalysators wird durch ein Glasfaserfilter filtriert und das Filtrat unter 11 Torr bei 40 eingedampft. Der Rückstand wird mit 500 ml Ether-Petrolether (1:1) verrührt. Die Suspension wird abfiltriert und die Krislalle mil 100 ml Ether-Petrolether gewaschen. Das 2-Amino-benzolsulfonamid schmilzt bei 152- 155°.

b) Eine Suspension von 102,8 g 2-Amino-benzolsulfonamid in 508 ml Wasser wird langsam mit 508 ml conc. Salzsäure vergetzl, anschliessend unter schnellem Rühren auf -5° abgekühlt und während 40 Minuten mit einer Lösung von 39,5g Natriumnitrit in 200 ml Wasser tropfenweise versetzt. Die gelbe Lösung wird während 1 Stunde bei 0° gerührt, mit 55 ml Methylenchlorid und anschliessend mit einer Lösung von 88,2 g Kaliumjodid in 103 ml Wasser tropfenweise versetzt. Die entstandene Suspension wird während 15 Stunden bei Raumtemperatur gerührt. Die organge-beigen Kristalle werden abfiltriert, mit 40 ml Wasser gewaschen und in 500 ml heissem Methanol gelöst. Unter Rühren wird die weisse Lösung mit 1000 ml Wasser versetzt und auf Raumtemperatur abgekühlt. Die ausgefallenen Kristalle werden abfiltriert und unter 0,1 Tor bei 25° gekocknet. Das 2-Jod-benzolsulfonamid schmilzt bei 168-169°.

c) Eine Mischung von 8,80 g 2-Jod-benzolsulfonamid, 0,60 g Bis-(Triphenylphosphin)-palladium(II)-dichlorid und 0,30 g Kupfer-(I)-jodid in 15 ml Dimethylformamid wird unter gutem Rühren mit 9,3 ml Triethylamin und 3,5 g Ethinyl-trimethylsilan versetzt. Die Mischung wird 3 Stunden bei 30° gerührt und auf 100 ml Eis/Wasser gegossen. Die Suspension wird dreimal mit je 100 ml Ethylacetat extrahiert. Die vereinigten organischen Phasen werden mit 50 ml Wasser und 50 ml Sole gewaschen, über Magnesiumsulfat getrocknet und unter 11 Torr bei 40° eingedampft. Der Rückstand chromatographiert man an 300 g Silicagel. Die Fraktionen 1-3, eluiert mit Ethylacetat-Hexan (1:1), werden verworfen. Die Fraktionen 4-7, eluiert mit den gleichen Gemischen, werden vereinigt und unter 0,1 Tor bei 40° eingedampft, wobei das 2-(2-Trimethylsilyl-ethinyl)-benzolsulfonamid kristallisiert. Die Kristalle werden abfiltriert. Smp. 76°.

d) Eine Lösung von 3,4 g 2-(2-Trimethylsilyl-ethinyl)-benzolsulfonamid in 10 ml Methanol wird unter leichter Kühlung und Rühren zu 20 ml 1 N Natronlauge zugetropft. Die Mischung wird eine Stunde bei Raumtemperatur gerührt und mit 15 ml 2 N Salzsäure angesäuert. Die Suspension wird dreimal mit je 70 ml Ethylacetat extrahiert. Die vereinigten organischen Phasen werden mit 40 ml Wasser gewaschen, über Magnesiumsulfat getrocknet und unter 11 Torr bei 40° eingeengt. Die ausgeschiedenen farblosen Kristalle werden abfiltriert und 15 Stunden unter 0.1 Torr bei 40° getrocknet. Das 2-Ethinyl-benzolsulfonamid schmilzt bei 130°

Alternativ kann das Ausgangsmaterial 2-Ethinyl-benzolsulfonamid wie folgt hergestellt werden:

a) Eine Mischung von 11,8 g 2-Jod-benzolsulfonamid, 4,2 ml 2-Methyl-3-butin-2-ol und 50 ml Triethylamin in 200 ml Dimethylformamid wird bei Raumtemperatur unter Rühren mit 100 mg Kupfer(I)-jodid und 220 mg Bis-(triphenylphosphin)-palladium(II)-dichlorid versetzt. Die Mischung wird 13 Stunden bei 70° gerührt und unter 0,1 Torr bei 50° eingedampft. Der dunkle Rückstand wird in 100 ml Ethylacetat-Ethanol (1:1) heiss gelöst. Die heisse Lösung wird mit Aktivkohle gerührt, filtriert und abgekühlt. Nach dem Zusatz von Ether scheiden sich farblose Kristalle ab. Man filtriert ab und trocknet die Kristalle 15 Stunden unter 0,1 Torr bei 50°. Das 2-(3-Hydroxy-3-methyl-but-1-in-1-yl)-benzolsulfonamid schmilzt bei 201-202°.

b) Eine Lösung von 3,0 g 2-(3-Hydroxy-3-methyl-but-1-in-1-yl)-benzolsulfonamid in 40 ml Toluol wird bei Raumtemperatur unter Rühren mit 1,5 g pulverisiertem Kaliumhydroxid versetzt. Die Mischung wird dann 3 Stunden bei 70° gerührt, abgekühlt und auf eine Mischung von 50 ml Eis-Wasser und 6 ml 2-n Salzsäure gegossen. Die organische Phase wird abgetrennt, mit 20 ml Wasser gewaschen, über Magnesiumsulfat getrocknet und unter 11 Torr bei 50° eingedampft. Den Rückstand kristallisiert man aus Ethylacetat. Das 2-Ethinyl-benzolsulfonamid schmilzt bei 130°.

Beispiel 4:

In analoger Weise wie in Beispiel 1 beschrieben wird hergestellt: N-[4-[5-(Cyclopentyloxycarbonyl-amino)-1-methyl-indol-3-yl-methyl]-3-methoxybenzoyl]-2-(prop-1-in-1-yl)-benzolsulfonamid, weisser amorpher Feststoff: $^1$H-NMR (400 MHz, DMSO-$d_6$): 8.89 (s, b, 1H); 8.05 (m, 1H); 7.57 (m, 2H); 7.53 (m, 3H); 7.43 (d,d, 1H); 7.24 (d, 1H); 7.16 (d, d, 1H); 7.14 (d, 1H); 6.97 (s, 1H); 5.08 (m, 1H); 3.96 (s, 2H); 3.92 (s, 3H); 3.68 (s, 3H); 2.04 (s, 3H); 1.90-1.55 (m, 8H);

ausgehend von 4-[5-(Cyclopentyloxycarbonyl-amino)-1-methyl-indol-3-yl-methyl]-3-methoxy-benzoesäure-N-succinimidester und 2-(Prop-1-in-1-yl)-benzolsulfonamid.

Beispiel 5:

In analoger Weise wie in Beispiel I beschrieben wird hergestellt: N-[4-[5-(Cyclopentyloxycarbonyl-amino)-1-methyl-indol-3-yl-methyl]-3-methoxybenzoyl]-2-(but-1-in-1-yl)-benzolsulfonamid, weisser amorpher Feststoff: $^1$H-NMR (400 MHz, DMSO-$d_6$): 8.85 (s, b, 1H); 8.0 (d, 1H); 7.58 (m, 2H); 7.45 (n, 4H); 7.23 (d, 1H); 7.16 (d, d, 1H); 7.05 (d, 1H); 6.91 (s, 1H); 5.08 (m, 1H); 3.94 (s, 2H); 3.88 (s, 3H); 3.67 (s, 3H); 2.34 (q, 2H); 1.06 (t, 3H); 1.90-1.54 (m, 8H);

ausgehend von 4-[5-(Cyclopentyloxycarbonyl-amino)-1-methyl-indol-3-yl-methyl]-3-methoxy-benzoesäure-N-succinimidester und 2-(But-1-in-1-yl)-benzolsulfonamid.

Das Ausgangsmaterial wird wie folgt hergestellt:

Zu einer Lösung von 5 g 2-Jod-benzolsulfonamid in 70 ml N,N'-Dimethylformamid werden 0,1 g Bis-(triphenylphosphin)-palladium-II-chlorid, 0,05 g Kupferjodid und 18.5 ml Triethylamin gegeben. Bei Raumtemperatur werden langsam Argon und 1-Butin eingeleitet. Nach 18 Stunden zeigt das Dünnschichtchromatogramm kein 2-Jod-benzolsulfonamid mehr. Das Dimethylformamid wird bei 0,1 Torr und 40° abgedampft und der Rückstand auf 300 g Kieselgel (Merck ST 60, 40-63 μm chromatographiert. Als Laufmittel dient Methylenchlorid. Auf diese Weise wird nach dem Eindampfen der das Produkt enthaltenen Fraktionen bei 15 Torr und 40° weisses pulverförmiges 2-(But-1-in-1-yl)-benzolsulfonamid erhalten: $^1$H-NMR (300 MHz, $CDCl_3$): 8.0 (d, d, 1H); 7.35 (d, d, 1H); 7.29 (d, t, 1H); 7.23 (d, t, 1H); 5.13 (s, 2H); 2.32 (q, 2H); 1.18 (t, 3H).

Analog kann hergestellt werden:

2-[Prop-1-in-1-yl)-benzolsulfonamid, weisse Kristalle mit einem Schmelzpunkt von 158- 160°, ausgehend von 2-Jodbenzolsulfonamid und Propin-Gas.

Beispiel 6:

In analoger Weise wie in Beispiel 1 beschrieben wird hergestellt: N-[4-[5-(Cyclopentyloxycarbonyl-amino)-1-methyl-indol-3-yl-methyl]-3-methoxybenzoyl]-2-(pent-1-in-1-yl)-benzolsulfonamid, weisser amorpher Feststoff: $^1$H-NMR (400 MHz, DMSO-$d_6$): 8.76 (s, b, 1H); 8.00 (d, 1H); 7.56 (m, 2H); 7.44 (m, 4H); 7.21 (d, 1H); 7.16 (d, d, 1H); 7.08 (d, 1H); 6.91 (s, 1H); 5.07 (m, 1H); 3.94 (s, 2H); 3.88 (s, 3H); 3.66 (s, 3H); 2.33 (t, 2H); 1.90-1.53 (m, 8H); 1.47 (m, 2H); 0.89 (t, 3H);
ausgehend von 4-[5-(Cyclopentyloxycarbonyl-amino)-1-methyl-indol-3-yl-methyl]3-methoxy-benzoesäure-N-succinimidester und 2-(Pent-1-in-1-yl )-benzolsulfonamid.

Beispiel 7:

In analoger Weise wie in Beispiel 1 beschrieben wird hergestellt N-[4-[5-(Cyclopentyloxycarbonyl-amino)-1-methyl-indol-3-yl-methyl]-3-methoxybenzoyl]-2-(3-methyl-but-1-in-1-yl)-benzolsulfonamid, weisser amorpher Feststoff: $^1$H-NMR (400 MHz, DMSO-$d_6$): 8.78 (b, 1H); 8.03 (n, 1H); 7.58 (m, 2H); 7.54-7.44 (n, 4H); 7.23 (d, 1H); 7.18 (d, d, 1H); 7.13 (d, 1H); 6.93 (s, 1H); 5.09 (m, 1H); 3.97 (s, 2H); 3.90 (s, 3H); 3.68 (s, 3H); 2.77 (m, 1H); 1.91-1.56 (m, 8H); 1.14 (d, 6H);
ausgehend von 4-[5-(Cyclopentyloxycarbonyl-amino)-1-methyl-indol-3-yl-methyl]-3-methoxy-benzoesäure-N-succinimidester und 2-(3-Methyl-but-1-in-1-yl)-benzolsulfonamid.

Beispiel 8:

In analoger Weise wie in Beispiel 1 beschrieben wird hergestellt: N-[4-[5-(Cyclopentyloxycarbonyl-amino)-1-methyl-indol-3-yl-methyl]-3-methoxy-benzoyl]-2-(5-acetyloxy-pent-1-in-1-yl)-benzolsulfonamid, weisser amorpher Feststoff: $^1$H-NMR (400 MHz), DMSO-$d_6$): 8.76 (s, b, 1H); 8.02 (d, b, 1H); 7.58 (d, 1H); 7.56 (d, 1H); 7.53-7.45 (m, b, 3H); 7.44 (d, d, 1H); 7.23 (d, 1H); 7.17 (d, d, 1H); 7.12 (d, 1H); 6.93 (s, 1H); 5.09 (m, 1H); 4.07 (t, 2H); 3.96 (s, 2H); 3.90 (s, 3H); 3.68 (s, 3H); 2.46 (t, 2H); 1.95 (s, 3H); 1.91-1.55 (m, 8H); 1.78 (m, 2H);
ausgehend von 4-[5-(Cyclopentyloxycarbonyl-amino)-1-methyl-indol-3-yl-methyl]-3-methoxy-benzoesäure-N-succinimidester und 2-(5-Acetyloxy-pent-1-in-1-yl)-benzolsulfonamid.

Das Ausgangsmaterial sowie analoges Ausgangsmaterial kann wie folgt hergestellt werden:

In 200 ml Benzol werden 3,0 g 2-(5-Hydroxypent-1-in-1-yl)-benzolsulfonamid und 4.7 ml Pyridin bei 45° gelöst. Die Lösung wird auf Raumtemperatur abgekühlt und 1.23 ml Acetylchlorid sowie 3,2 ml Triethylamin zugegeben. Unter Argon wird 1 Stunde bei Raumtemperatur gerührt, bis das Dünnschichtchromatogramm kein Ausgangsmaterial mehr zeigt. Das Lösungsmittel wird bei 15 Torr und 40° abgedampft. Der Rückstand wird mit einer Mischung aus Essigester, Wasser und 1n Salzsäure (100 ml) versetzt. Im Scheidetrichter wird die organische Phase nach dem Schütteln abgetrennt und mit 0,1n Salzsäure sowie mit gesättigter Kochsalzlösung gewaschen, getrockent (über Magnesiumsulfat) und bei 15 Torr und 40° eingedampft. Das Rohprodukt wird auf 200 g Kieselgel (Merck, ST 60, 40-63 μm) mit Hexan/Ethylacetat = 1/1 als Laufmittel gereinigt. Auf diese Weise erhält man 2-(5-Acetyloxy-pent-1-in-1-yl)-benzolsulfonamid als weisse Kristalle. Der Schmelzpunkt beträgt 66-68°. $^1$H-NMR (300 MHz), DMSO-$d_6$): 7.87 (d, d, 1H); 7.45-7.60 (m, 3H); 7.20 (s, 2H); 4.13 (t, 2H); 2.57 (t, 2H); 2.02 (S, 3H); 1.86 (m, 2H).

Analog können hergestellt werden:

2-(3-Acetyloxy-prop-1-in-1-yl)-benzolsulfonamid, weisse Kristalle, Schmelzpunkt 136-138°, ausgehend von 2-(3-Hydroxy-prop-1-in-1-yl)-benzolsulfonamid.

2-(4-Acetyloxy-but-1-in-1-yl)-benzolsulfonamid, farbloses Oel. $^1$H-NMR (200 MHz), $CDCl_3$): 4.35 (t, 2H); 2.87 (t, 2H); 2.15 (s, 3H); ausgehend von 2-(4-Hydroxy-but-1-in-1-yl-benzolsulfonamid.

Beispiel 9:

In analoger Weise wie in Beispiel 1 beschrieben wird hergestellt: N-[4-[5-(Cyclopentyloxycarbonyl-amino)-1-methyl-indol-3-yl-methyl]-3-methoxybenzoyl]-2-(4-acetyloxy-but-1-in-1-yl)-benzolsulfonamid, weisser amorpher Feststoff: $^1$H-NMR (400 MHz, DMSO-$d_6$): 8.88 (s, b, 1H); 8.0 (m, 1H); 7.59 (d, 1H); 7.57 (d, 1H); 7.44 (m, 4H); 7.23 (d, 1H); 7.17 (d, d, 1H); 7.07 (d, 1H); 6.92 (s, 1H); 5.08 (m, 1H); 4.11 (t, 2H); 3.94 (s, 2H); 3.88 (s, 3H); 3.68 (s, 3H); 2.69 (t, 2H); 1.96 (s, 3H); 1.91-1.56 (m, 8H);
ausgehend von 4-[5-(Cyclopentyloxycarbonyl-amino)-1-methyl-indol-3-yl-methyl]-3-methoxy-benzoesäure-N-succinimidester und 2-(4-Acetyloxy-but-1-in-1-yl)-benzolsulfonamid

Beispiel 10:

In analoger Weise wie in Beispiel 1 beschrieben wird hergestellt: N-[4-[5-(Cyclopentyloxycarbonyl-amino)-1-methyl-indol-3-yl-methyl]-3-methoxy-benzoyl]-2-(3-acetyloxy-prop-1-in-1-yl)-benzolsulfonamid, weisser amorpher Feststoff: $^1$H-NMR (400 MHz, DMSO-$d_6$): 8.89 (s, b, 1H); 8.09 (m, 1H); 7.62 (m, 3H); 7.58 (d, 1H); 7.54 (d, 1H); 7.4.3 (d, d, 1H); 7.24 (d, 1H); 7.16 (d, d, 1H); 7.13 (d, 1H); 6.97 (s, 1H); 5.08 (m, 1H); 4.92 (s, 2H); 3.97 (s, 2H); 3.92 (s, 3H); 3.69 (s, 3H); 1.95 (s, 3H); 1.91-1.55 (m, 8H);
ausgehend von 4-[5-(Cyclopentyloxycarbonyl-amino)-1-methyl-indol-3-yl-methyl]-3-methoxy-benzoesäure-N-succinimidester und 2-(3-Acetyloxy-prop-1-in-1-yl)-benzolsulfonamid.

Beispiel 11:

In analoger Weise wie in Beispiel 1 beschrieben wird hergestellt: N-[4-[5-(Cyclopentyloxycarbonyl-amino)-1-methyl-indol-3-yl-methyl]-3-methoxy-benzoyl]-2-(4-acetyloxy-pent-1-in-1-yl)-benzolsulfonamid, weisser amorpher Feststoff: $^1$H-NMR (400 MHz), DMSO-$d_6$); 8.76 (b, 1H); 7.98 (m, 1H); 7.59 (b, 2H); 7.46 (d, d, 1H); 7.37 (m, 3H); 7.22 (d, 1H); 7.18 (d, d, 1H); 7.04 (d, 1H); 6.89 (s, 1H); 5.09 (m, 1H); 4.91 (m, 1H); 3.94 (s, 2H); 3.86 (s, 3H); 3.67 (s, 3H); 2.61 (m, 2H); 1.95 (s, 3H); 1.92-1.55 (m, 8H); 1.31 (d, 3H);
ausgehend von 4-[5-(Cyclopentyloxycarbonyl-amino)-1-methyl-indol-3-yl-methyl]-3-methoxy-benzoesäure-N-succinimidester und 2-(4-Acetyloxy-pent-1-in-1-yl)-benzolsulfonamid.

Das Ausgangsmaterial sowie analoges Ausgangsmaterial kann wie folgt hergestellt werden:

1 g 2-(4-Hydroxy-pent-1-in-1-yl)-benzolsulfonamid werden in 9 ml Pyridin gelöst, auf 5° abgekühlt und 0,44 ml Acetanhydrid zugegeben. Das Reaktionsgemisch wird bei 5° 14 Stunden und dann bei Raumtemperatur weite 24 Stunden unter Argon gerührt. Anschliessend wird das Reaktionsgemisch auf 100 ml eiskalte 1n Salzsäure gegossen und zweimal mit je 50 ml Essigester extrahiert. Die vereinigten organischen Phasen werden zweimal mit je 20 ml 1n Salzsäure, mit 50 ml Wasser und mit 30 ml gesättigter Kochsalzlösung gewaschen, über Magnesiumsulfat getrocknet, abfiltriert und bei 15 Torr und 40° eingedampft. Das Rohprodukt wird auf 80 g Kieselgel (Merck ST 60, 40-63 μm) chromatographiert. Als Laufmittel dient eine 1/1 -Mischung aus Hexan und Ethylacetat. Man erhält 2-(4-Acetyloxy-pent-1-in-1-yl)-benzolsulfonamid als farbloses Oel. $^1$H-NMR (200 MHz), DMSO-$d_6$): 7.90 (d, d, 1H); 7.48-7.63 (m, 3H); 7.22 (s, 2H); 5.01 (m, 1H); 2.82 (d, 2H); 2.05 (s, 3H); 1.33 (d, 3H).

Analog kann hergestellt werden:

2-(3-Acetyloxy-but-1-in-1-yl)-benzolsulfonamid, farbloses Oel. $^1$H-NMR (200 MHz), $CDCl_3$): 8.03 (d, d, 1H); 7.40-6.70 (m, 3H); 5.50 (s, 2H); 5.45 (q, 1H); 2.13 (s, 3H); 1.65 (d, 3H); ausgehend von 2-(3-Hydroxy-but-1-in-1-yl)-benzolsulfonamid.

Beispiel 12:

In analoger Weise wie in Beispiel 1 beschrieben wird hergestellt: N-[4-[5-(Cyclopentyloxycarbonyl-amino)-1-methyl-indol-3-yl-methyl]-3-methoxy-benzoyl]-2-(5-pivaloyloxy-pent-1-in-1-yl)-benzolsulfonamid, weisser amorpher Feststoff: $^1$H-NMR (400 MHz, DMSO-$d_6$): 8.00 (m, 1H); 7.58 (d, 1H); 7.57 (d, 1H); 7.46 (d, d, 1H); 7.41 (m, 3H); 5.09 (m, 1H); 4,11 (t, 2H); 3.95 (s, 2H); 3.87 (s, 3H); 3.68 (s, 3H); 2.43; ausgehend von 4-[5-(Cyclopentyloxycarbonyl-amino)-1-methyl-indol-3-yl-methyl]-3-methoxy-benzoesäure-N-succinimidester und 2-(5-Pivaloyloxy-pent-1-in-1-yl)-benzolsulfonamid.

Das Ausgangsmaterial wird wie folgt hergestellt:

In 200 ml Benzol werden 2,0 g 2-(5-Hydroxy-pent-1-in-1-yl)-benzolsulfochlorid und 3,13 ml Pyridin, bei 45° gelöst. Die Lösung wird auf Raumtemperatur abgekühlt und 1,41 ml Pivalinsäurechlorid, sowie 2,15 ml Triethylamin zugegeben. Unter Argon wird 0,5 Stunden bei Raumtemperatur gerührt, bis das Dünnschichtchromatogramm kein Ausgangsmaterial mehr zeigt. Das Lösungsmittel wird bei 15 Torr und 40° abgedampft. Der Rückstand wird mit einer Mischung aus Essigester, Wasser und 1n Salzsäure versetzt. Im Scheidetrichter wird die organische Phase nach dem Schütteln abgetrennt und mit 0,1n Salzsäure sowie mit gesättigter Kochsalzlösung gewaschen, über Magnesiumsulfat getrocknet und bei 15 Torr und 40° eingedampft. Das Rohprodukt wird auf 200 g Kieselgel (Merck, ST 60, 40-63 μm) mit Hexan/Ethylacetat = 1/1 als Laufmittel sereinigt. Auf diese Weise werden 1,0 g 2-(5-Pivaloyloxy-pent-1-in-1-yl-benzolsulfonamid in Form eines gelblichen Oels erhalten. $^1$H-NMR (400 MHz), DMSO-$d_6$): 7.88 (d, d, 1H); 7.47-7.60 (m, 3H); 7.18 (s, 2H); 4.16 (t, 2H); 2.57 (6, 2H); 1.90 (m, 2H); 1.08 (s, 9H).

Analog können hergestellt werden:

2-(4-Pivaloyloxy-but-1-in-1-yl)-benzolsulfonamid, weisse Kristalle vom Schmelzpunkt 79-80°C. $^1$H-NMR (200 MHz), DMSO-$d_6$): 7.90 (d, d, 1H); 7.48-7.62 (m, 3H); 7.22 (s, 2H); 4.22 (t, 2H); 2.86 (t, 2H); 1.08 (s,

9H).

2-(3-Pivaloyloxy-but-1-in-1-yl)-benzolsulfonamid, gelbe Kristalle $^1$H-NMR (200 MHz), DMSO-$d_6$): 7.88 (d, d, 1H); 7.51-7.70 (m, 3H); 7.23 (s, 2H); 5.00 (s, 2H) 1.20 (s, 9H).

Beispiel 13:

In analoger Weise wie in Beispiel 1 beschrieben wird hergestellt: N-[4-[5-(Cyclopentyloxycarbonyl-amino)-1-methyl-indol-3-yl-methyl]-3-methoxy-benzoyl]-2-(4-pivaloyloxy-but-1-in-1-yl)-benzolsulfonamid, weisser amorpher Feststoff: $^1$H-NMR (400 MHz, DMSO-$d_6$): 9.0 (b, 1H); 8.02 (d, b, 1H): 7.59 (d, 1H); 7.56 (d, 1H); 7.48 (m, b, 3H); 7.43 (d, d, 1H); 7.24 (d, 1H); 7.16 (d, d, 1H); 7.08 (d, b, 1H); 6.95 (s, 1H); 5.07 (m, 1H); 4.11 (t, 2H); 3.93 (s, 2H); 3.89 (s, 3H); 3.68 (s, 3H); 2.72 (t,b, 2H); 1.90-1.54 (m, 8H); 1.10 (s. 9H); ausgehend von 4-[5-(Cyclopentyloxycarbonyl-amino)-1-methyl-indol-3-yl-methyl]-3-methoxy-benzoesäure-N-succinimidester und 2-(4-Pivaloyloxy-but-1-in-1-yl)-benzolsulfonamid.

Beispiel 14:

In analoger Weise wie in Beispiel 1 beschrieben wird hergestellt: N-[4-[5-(Cyclopentyloxycarbonyl-amino)-1-methyl-indol-3-yl-methyl]-3-methoxy-benzoyl]-2-(3-pivaloyloxy-prop-1-in-1-yl)-benzolsulfonamid, weisser amorpher Feststoff: $^1$H-NMR (400 MHz, DMSO-$d_6$): 8.89 (b, 1H); 8.08 (m, 1H); 7.60 (m, b, 3H); 7.58 (d, 1H); 7.54 (d, 1H); 7.43 (d, d, 1H); 7.24 (d, 1H); 7.17 (d, d, 1H); 7.12 (d, 1H); 6.97 (s, 1H); 5.08 (m, 1H); 4.93 (s, 2H); 3.96 (s, 2H); 3.91 (s, 3H); 3.68 (s, 3H); 1.90-1.55 (m, 9H); 1.11 (s, 9H); ausgehend von 4-[5-(Cyclopentyloxycarbonyl-amino)-1-methyl-indol-3-yl-methyl]-3-methoxy-benzoesäure-N-succinimidester und 2-(3-Pivaloyloxy-prop-1-in-1-yl)-benzolsulfonamid.

Beispiel 15:

In analoger Weise wie in Beispiel 1 beschrieben wird hergestellt: N-[4-[5-(Cyclopentyloxycarbonyl-amino)-1-methyl-indol-3-yl-methyl]-3-methoxy-benzoyl]-2-(3-hydroxy-3-methyl-but-1-in-1-yl)-benzolsulfonamid, weisser amorpher Feststoff: $^1$H-NMR (400 MHz), DMSO-$d_6$); 8.74 (s, b, 1H); 8.03 (d, d, 1H); 7.57 (d, d, 1H); 7.55 (d, 1H); 7.53 (d, 1H); 7.54-7.44 (m, 2H); 7.42 (d, d, 1H); 7.21 (d, 1H); 7.16 (d, d, 1H); 7.10 (d, 1H); 6.91 (s, 1H); 5.08 (m, 1H); 3.95 (s, 2H); 3.88 (s, 3H); 3.66 (s, 3H); 1.91-1.52 (m, 8H); 1.41 (s, 6H); ausgehend von 4-[5-(Cyclopentyloxycarbonyl-amino)-1-methyl-indol-3-yl-methyl]-3-methoxy-benzoesäure-N-succinimides ter und 2-(3-Hydroxy-3-methyl-but-1-in-1-yl)-benzolsulfonamid.

Beispiel 16:

In analoger Weise wie in Beispiel 1 beschrieben wird hergestellt: N-[4-[5-(Cyclopentyloxycarbonyl-amino )-1-methyl-indol-3-yl-methyl]-3-methoxy-benzoyl]-2-(.3-methoxy-prop-1-in-1-yl)-benzolsulfonamid, weisser amorpher Feststoff: $^1$H-NMR (400 MHz), DMSO-$d_6$): 8.88 (s, b, 1H); 7.98 (m, 1H); 7.58 (d, 1H); 7.57 (d, 1H); 7.44 (d, d, 1H); 7.40 (m, 3H); 7.23 (d, 1H); 7.17 (d, d, 1H); 7.02 (d, 1H); 6.91 (s, 1H); 5.09 (m, 1H); 4.21 (s, 2H); 3.93 (s, 2H); 3.86 (s, 3H); 3.68 (s, 3H); 3.29 (s, 3H); 1.91-1.55 (m, 8H); ausgehend von 4-[5-(Cyclopentyloxycarbonyl-amino)-1-methyl-indol-3-yl-methyl]-3-methoxy-benzoesäure-N-succinimidester und 2-(3-Methoxy-prop-1-in-1-yl)-benzolsulfonamid.

Beispiel 17:

In analoger Weise wie in Beispiel 1 beschrieben wird hergestellt: N-[4-[5-(Cyclopentyloxycarbonyl-amino)-1-methyl-indol-3-yl-methyl]-3-methoxy-benzoyl]-2-(4-methoxy-but-1-in-1-yl)-benzolsulfonamid, weisser amorpher Feststoff: $^1$H-NMR (400 MHz), DMSO-$d_6$): 8.76 (b, 1H); 8.06 (m, 1H); 7.58 (d, 1H); 7.54 (d, 1H); 7.60-7.50 (m, 3H); 7.43 (d, d, 1H); 7.23 (d, 1H); 7.17 (d, d, 1H); 7.15 (d, 1H); 6.95 (s, 1H); 5.09 (m, 1H); 3.98 (s, 2H); 3.92 (s, 3H); 3.68 (s, 3H); 3.44 (t, 2H); 3.20 (s, 3H); 2.64 (t, 2H); 1.92-1.55 (m, 8H); ausgehend von 4-[5-(Cyclopentyloxycarbonyl-amino)-1-methylindol-3-yl-methyl]-3-methoxy-benzoesäure-N-succinimidester und 2-(4-Methoxy-but-1-in-1-yl)-benzolsulfonamid.

Beispiel 18:

Zu einer Lösung von 1,43 g N-[4-(5-Amino-1-methyl-indol-3-ylmethyl)-3-methoxy-benzoyl]-2-(5-methoxy-pent-1-in-1-yl)-benzolsulfonamid in 10 ml Dichlormethan und 0,78 g N-Methylmorpholin setzt man eine

Lösung von 0,39 g Chlorameisensäurecyclopentylester in 20 ml Dichlormethan zu. Die Mischung wird drei Stunden unter Einleiten von Stickstoff bei Raumtemperatur gerührt und mit 10 ml 1-n Salzsäure und 10 ml Wasser gewaschen. Die organische Phase wird abgetrennt, über Magnesiumsulfat getrocknet und unter 11 Torr bei 40° eingedampft. Der Rückstand, ein gelber Schaum, wird an 100 g Silicagel flash-chromatographiert. Die Fraktionen 1-5, eluiert mit je 50 ml Hexan-Ethylacetat (1:2), werden verworfen. Die Fraktionen 6-9, eluiert mit je 50 ml Ethylacetat, werden vereinigt und unter 11 Torr bei 40° eingedammpft. Das N-[4-[5-(Cyclopentyloxycarbonyl-amino)-1-methyl-indol-3-yl-methyl]-3-methoxy-benzoyl]-2-(5-methoxy-pent-1-in-1-yl)-benzolsulfonamid liegt als gelbliches Pulver vor. $^1$H-NMR (400 MHz, DMSO-$d_6$): 8.88 (s, b, 1H); 7.95 (m, 1H); 7.59 (d, 1H); 7.58 (d, 1H); 7.45 (d, b, 1H); 7.34 (m, b, 3H); 7.23 (d, 1H); 7.17 (d, d, 1H); 7.02 (d, 1H); 6.91 (s, 1H); 5.09 (m, 1H); 3.93 (s, 2H); 3.86 (s, 3H); 3.67 (s, 3H); 3.39 (t, 2H); 3.18 (s, 3H); 2.35 (t, 2H); 1.92-1.55 (m, 8H); 1.67 (m, 2H).

Das Ausgangsmaterial wird wie folgt hergestellt:

a) Eine Mischung von 3,4 g N-(3-Dimethylaminopropyl)-N′-ethyl-carbodiimid-hydrochlorid, 3,95 g 2-Jod-benzolsulfonamid und 2,15 g 4-Dimethylaminopyridin in 150 ml Dichlormethan wird unter Rühren und Einleiten von Argon bei Raumtemperatur mit einer Lösung von 5,0 g 3-Methoxy-4-(1-methyl-5-nitro-indol-3-yl-methyl)-benzoesäure in 250 ml Tetrahydrofuran versetzt. Die Mischung wird 5 Stunden bei 42° und anschliessend 15 Stunden bei Raumtemperatur gerührt. Die ausgeschiedenen gelben Kristalle werden abfiltriert und in 200 ml Trichlormethan/Methanol (9:1) unter Erwärmen gelöst und die Lösung an 500 g Silicagel flash-chromatographiert. Die Fraktionen 1 und 2, eluiert mit je 400 ml Trichlormethan-Methanol (9:1), werden verworfen. Die Fraktionen 3-7, eluiert mit je 400 ml desselben Gemisches, werden vereinigt und unter 11 Torr bei 40° eingedampft. Den Rückstand verrührt man mit Diethylether, filtriert ab und trocknet die gelben Kristalle 20 Stunden bei 40°. Das N-[4-(1-Methyl-5-nitro-indol-3-yl-methyl)-3-methoxy-benzoyl]-2-jod-benzolsulfonamid schmilzt bei 180-230° (unter Zersetzung). $^1$H-NMR (400 MHz, DMSO-$d_6$): 8,51 (d, 1H); 8,07 (d, d, 1H); 8.00 (d, d, 1H); 7,89 (d, d, 1H); 7,61 (d, 1H); 7,55 (d, 1H); 7,47 (d, d, 1H); 7,42 (t, d, 1H); 7,28 (s, 1H); 7,10 (d, 1H); 7,06 (t, d, 1H); 4,05 (s, 2H); 3,87 (s, 3H); 3,80 (s, 3H).

b) Eine Mischung von 1,21 g N-[4-(1-Methyl-5-nitro-indol-3-yl-methyl)-3-methoxy-benzoyl]-2-jodbenzolsulfonamid, 0,8 g einer 60%igen Lösung von Pent-4-in-1-yl-methylether in Ether-Tetrahydroturan (1:1) und 2,5 ml Triethylamin in 10,2 ml Dimethylformamid wird bei Raumtemperatur unter Rühren mit 20 mg Kupter(I)-jodid und 40 mg Bis-(triphenylphosphin)-palladium (II)-dichlorid versetzt. Die Mischung wird 15 Stunden bei 70° in einem kleinen Bombenrohr gerührt und unter 0,1 Torr bei 40° eingedampft. Der Rückstand wird auf 35 g Silicagel flash-chromatographiert. Die Fraktionen 1-7, eluiert mit je 20 ml Ethylacetat, werden verworfen. Die Fraktionen 8-17, eluiert mit je 20 ml Ethylacetat, werden vereinigt und unter 11 Torr bei 40° eingedampft. Der Rückstand, das N-[4-(5-nitro-1-methyl-indol-3-yl-methyl)-3-methoxy-benzoyl]-2-(5-methoxy-pent-1-in-1-yl)-benzolsulfonamid liegt als gelbes Pulver vor. $^1$H-NMR (400 MHz, DMSO-$d_6$): 8,49 (d, 1H); 8,00 (d, d, 1H); 7,98 (m, b, 1H); 7,60 (d, 1H); 7,55 (d, 1H); 7,48 (d, d, 1H); 7,40 (m, b, 3H); 7,28 (s, 1H); 7,15 (d, b, 1H); 4,08 (s, 2H); 3,89 (s, 3H); 3,81 (s, 3H); 3,34 (t, 2H); 3,15 (s, 3H); 2,37 (t, 2H); 1,64 (m, 2H).

Alternativ kann man das N-[4-(5-nitro-1-methyl-indol-3-yl-methyl)-3-methoxybenzoyl]-2-(5-methoxy-pent-1-in-1-yl)-benzolsulfonamid wie folgt herstellen:

Eine Lösung von 2,18 g 3-Methoxy-4-(1-methyl-5-nitro-indol-3-ylmethyl)-benzoesäure-N-succinimidester, 1,40 g 2-(5-Methoxy-pent-1-in-1-yl)-benzolsulfonamid und 2,3 ml DBU in 50 ml Dichlormethan wird 15 Stunden unter Argon bei Raumtemperatur gerührt. Die Mischung wird mit 50 ml Dichlormethan verdünnt und zweimal mit je 20 ml 1n Salzsäure und mit 20 ml Wasser gewaschen, über Magnesiumsulfat getrocknet und unter 11 Torr bei 40° eingedampft. Der Rückstand wird an 100 g Silicagel flash-chromatographiert. Die Fraktionen 1 und 2, eluiert mit je 80 ml Ethylacetat, werden verworfen. Die Fraktionen 3-7, eluiert mit demselben Lösungsmittel, werden vereinigt und unter 11 Torr bei 50° eingedampft. Den Rückstand rührt man 30 Minuten mit 50 ml Ether/Petrolether (1:1), filtriert ab und trocknet den Rückstand 15 Stunden unter 0,1 Torr bei Raumtemperatur. Das N-[4-(5-Nitro-1-methyl-indol-3-yl-methyl)-3-methoxy-benzoyl]-2-(5-methoxy-pent-1-in-1-yl)-benzolsulfonamid liegt als gelbes Pulver vor.

c) Eine Suspension von 1,67 g N-[4-(5-Nitro-1-methyl-indol-3-yl-methyl)-3-methoxy-benzoyl]-2-(5-methoxy-pent-1-in-1-yl)-benzolsulfonamid in 60 ml Ethanol wird unter Rühren mit 3,25 g Zinn(II)-chlorid-dihydrat versetzt. Die Mischung wird 15 Stunden unter Rückfluss gerührt, wobei Lösung eintritt. Man engt unter 11 Torr bei 50° zur Trockene ein, verrührt den Rückstand mit 20 ml gesättigter wässriger Natriumbikarbonat-Lösung und extrahiert zweimal mit je 50 ml Ethylacetat. Die vereinigten organischen Phasen werden über Magnesiumsulfat getrocknet und unter 11 Torr bei 50° eingedampft. Der Rückstand wird an 60 g Silicagel flash-chromatographiert. Die Fraktionen 1-8, eluiert mit je 100 ml Ethylacetat, werden verworfen. Die Fraktionen 9-25, eluiert mit je 100 ml Ethylacetat-Isopropanol (9:1), werden vereinigt und unter 11 Torr bei 50° eingedampft. Der Rückstand, das N-[4-(5-Amino-1-methyl-indol-3-yl-methyl)-3-methoxy-benzoyl]-2-(5-methoxy-pent-1-in-1-yl)-benzolsulfonamid, liegt als gelber Schaum vor. $^1$H-NMR (100 MHz, DMSO-$d_6$): 7,91 (m, 1H); 7,53 (s, 1H); 7,32 (m, 3H); 7,07 (d, 1H); 6,93 (d, 1H); 6,60 (s, 1H); 6,60 (d, 1H); 6,54 (dd, 1H); 3,83 (s, 5H); 3,61 (s, 3H); 3,36 (t, 2H); 3,14 (s, 3H); 2,33 (t, 2H); 1,62 (m, 2H).

Beispiel 19: N-[4-[5-(Cyclopentyloxycarbonyl-amino)-1-methyl-indol-3-yl-methyl]-3-methoxy-benzoyl]-2-(5-hydroxy-pent-1-in-1-yl)-benzolsulfonamid

Zu einer Lösung von 1,2 g N-[4-[5-(Cyclopentyloxycarbonyl-amino)-1-methyl-indol-3-yl-methyl]-3-methoxy-benzoyl]-2-(5-acetyloxy-pent-1-in-1-yl)-benzolsulfonamid in 40 ml Methanol/Tetrahydrofuran = 1/1 gibt man 0,37 g Lithiumhydroxid-monohydrat und 10 ml Wasser. Die Mischung wird 16 Stunden bei Raumtemperatur gerührt, dann werden die Lösungsmittel bei 15 Torr und 40° abgedampft und der Rückstand mit 50 ml Methylenchlorid versetzt. Durch Zugabe von 50 ml 1n Salzsäure wird angesäuert. Im Scheidetrichter wird die organische Phase abgetrennt. Die wässrige Phase wird noch zweimal mit je 20 ml Methylenchlorid extrahiert. Die vereinigten organischen Phasen werden mit halbgesättigter Kochsalzlösung gewaschen, über Magnesiumsulfat getrocknet und bei 15 Torr und 40° eingedampft. Der Rückstand wird auf 45 g Kieselgel (Merck, ST 60, 4-63 μm) mit 20 % Hexan in Ethylacetat chromatographiert. Auf diese Weise werden 1,1 g der Titelverbindung als weisser amorpher Feststoff erhalten. $^1$H-NMR (400 MHz, DMSO-$d_6$): 8,90 (s, b, 1H); 8,05 (m, 1H); 7,56 (m, 5H); 7,43 (d, d, 1H); 7,24 (d, 1H); 7,17 (d, d, 1H); 7,15 (d, 1H); 6,96 (s, 1H); 5,08 (m, 1H); 3,97 (s, 2H); 3,92 (s, 3H); 3,68 (s, 3H); 3,47 (t, 2H); 2,47 (t, 2H); 1,92-1,55 (m, 8H); 1,64 (m, 2H).

Beispiel 20:

In analoger Weise wie in Beispiel 19 beschrieben wird hergestellt: N-[4-[5-(Cyclopentyloxycarbonyl-amino)-1-methyl-indol-3-yl-methyl]-3-methoxy-benzoyl]-2-(4-hydroxy-but-1-in-1-yl)-benzolsulfonamid, weisser Feststoff: $^1$H-NMR (400 MHz, DMSO-$d_6$): 8,75 (s, b, 1H); 8,01 (m, 1H); 7,59 (d, 1H); 7,57 (d, 1H); 7,42 (d, d, 1H); 7,40 (m, 3H); 7,22 (d, 1H); 3,94 (s, 2H); 3,88 (s, 3H); 3,67 (s, 3H); 3,60 (t, 2H); 2,50 (t, 2H); 1,91-1,55 (m, 8H);
ausgehend von N-[4-[5-(Cyclopentyloxycarbonyl-amino)-1-methyl-indol-3-yl-methyl]-3-methoxy-benzoyl]-2-(4-acetyloxy-but-1-in-1-yl)-benzolsulfonamid.

Beispiel 21:

In analoger Weise wie in Beispiel 19 beschrieben wird hergestellt: N-[4-[5-(Cyclopentyloxycarbonyl-amino)-1-methyl-indol-3-yl-methyl]-3-methoxy-benzoyl]-2-(3-hydroxy-prop-1-in-1-yl)-benzolsulfonamid, weisser amorpher Feststoff: $^1$H-NMR (400 MHz, DMSO-$d_6$): 8,88 (b, 1H); 7,98 (m, b, 1H); 7,58 (d, 1H); 7,57 (d, 1H); 7,42 (d, d, 1H); 7,40 (m, 3H); 7,23 (d, 1H); 7,18 (d, d, 1H); 7,03 (d, b, 1H); 6,91 (s, 1H); 5,09 (m, 1H); 4,24 (s, 2H); 3,93 (s, 2H); 3,86 (s, 3H); 3,68 (s, 3H); 1,91-1,55 (m, 8H); ausgehend von N-[4-[5-(Cyclopentyloxycarbonyl-amino)-1-methyl-indol-3-yl-methyl]-3-methoxy-benzoyl]-2-(3-acetyloxy-prop-1-in-1-yl)-benzolsulfonamid.

Beispiel 22:

In analoger Weise wie in Beispiel 19 beschrieben wird hergestellt: N-[4-[5-(Cyclopentyloxycarbonyl-amino)-1-methyl-indol-3-yl-methyl]-3-methoxy-benzoyl]-2-(3-hydroxy-but-1-in-1-yl)-benzolsulfonamid, weisser amorpher Feststoff: $^1$H-NMR (400 MHz, DMSO-$d_6$): 8,88 (s, b, 1H); 8,07 (d, b, 1H); 7,58 (m, 5H); 7,43 (d, d, 1H); 7,24 (d, 1H); 7,17 (d, d, 1H); 7,13 (d, 1H); 6,95 (s, 1H); 5,08 (m, 1H); 4,61 (q, 1H); 3,96 (s, 2H); 3,91 (s, 3H); 3,68 (s, 3H); 1,91-1,55 (m, 8H); 1,31 (d, 3H); ausgehend von 2-(3-Acetyloxy-but-1-in-1-yl)-benzolsulfonamid (Beispiel 11) über das N-[4-[5-(Cyclopentyloxycarbonyl-amino)-1-methyl-indol-3-yl-methyl]-3-methoxybenzoyl-2-(3-acetyloxy-but-1-in-1-yl)-benzolsulfonamid.

Beispiel 23:

In analoger Weise wie in Beispiel 19 beschrieben wird hergestellt: N-[4-[5-(Cyclopentyloxycarbonyl-amino)-1-methyl-indol-3-yl-methyl]-3-methoxy-benzoyl]-2-(4-hydroxy-pent-1-in-1-yl)-benzolsulfonamid, weisser amorpher Feststoff: $^1$H-NMR (400 MHz, DMSO-$d_6$): 8,76 (b, 1H); 8,02 (m, 1H); 7,58 (b, 2H); 7,45 (m, 3H); 7,42 (d, d, 1H); 7,22 (d, 1H); 7,08 (d, 1H); 6,92 (s, 1H); 5,09 (m, 1H); 3,95 (s, 2H); 3,90 (s, 3H); 3,88 (m, 2H); 3,68 (s, 3H); 2,44 (m, 2H); 1,91-1,55 (m, 8H); 1,14 (d, 3H); ausgehend von N-[4-[5-(Cyclopentyloxycarbonyl-amino)-1-methyl-indol-3-yl-methyl]-3-methoxybenzoyl]-2-(4-acetoxy-pent-1-in-1-yl)-benzolsulfonamid.

Beispiel 24: N-[4-[5-(Cyclopentyloxycarbonyl-amino)-1-methyl-indol-3-yl-methyl]-3-methoxy-benzoyl]-2-(3-acetyloxy-3-methyl-but-1-in-1-yl)-benzolsulfonamid

Zu einer Lösung von 1,0 g N-[4-[5-(Cyclopentyloxycarbonylamino)-1-methyl-indol-3-yl-methyl]-3-methoxy-benzoyl]-2-(3-hydroxy-3-methyl-but-1-in-1-yl)-benzolsulfonamid in 6 ml Pyridin werden 0,44 g Essigsäureanhydrid und 0,1 g 4-Dimethylaminopyridin gegeben. Die Reaktionslösung wird 20h bei Raumtemperatur unter Argon gerührt. Anschliessend wird das Reaktionsgemisch mit 100 ml Ethylacetat verdünnt. Die Ethyl-Acetatphase wird zweimal mit je 80 ml 1n Salzsäure, mit 50 ml Wasser und mit 50 ml gesättigter Kochsalzlösung gewaschen. Ueber Magnesiumsulfat wird die Ethylacetatphase getrocknet und bei 20 Torr und 40° eingedampft. Der Rückstand wird auf 50 g Silicagel (Merck, ST 60, 40-63 μm) mit Hexan/Ethylacetat = 1/1 chromatographiert. Auf diese Weise erhält man die Titelverbindung als weissen amorphen Feststotf. $^1$H-NMR (400 MHz, DMSO-$d_6$): 8,87 (b, 1H); 8,06 (d, d, 1H); 7,61 (m, 3H); 7,55 (d, 1H); 7,50 (d, 1H); 7,41 (d, d, 1H); 7,22 (d, 1H); 7,14 (d, d, 1H); 7,13 (d, 1H); 6,94 (s, 1H); 5,06 (m, 1H); 3,95 (s, 2H); 3,90 (s, 3H); 3,66 (s, 3H); 1,91 (s, 3H); 1,89-1,52 (m, 8H); 1,61 (s, 6H).

Beispiel 25: N-[4-[5-(Cyclopentyloxycarbonyl-amino)-1-methyl-indiol-3-yl-methyl]-3-methoxy-benzoyl]-2-(pent-1-in-1-yl)-benzolsulfonamid

Zu einer Lösung von 2,0 g 4-[5-(Cyclopentyloxycarbonylamino)-1-methyl-indol-3-yl-methyl]-3-methoxy-benzoesäure in 50 ml Methylenchlorid werden 1,07 g 2-(Pent-1-in-1-yl)-benzolsulfonamid und 0,94 g 3-[N-Dimethyl-aminopropyl]-N-ethylcarbodiimidhydrochlorid, sowie 0,6 g 4-Dimethylaminopyridin zugegeben. Diese Reaktionsmischung wird während 20 Stunden unter Argon gerührt. Anschliessend wird das Reaktionsgemisch mit 50 ml Methylenchlorid verdünnt. Die Methylenchloridphase wird im Scheidetrichter zweimal mit 1n Salzsäure und einmal mit Wasser gewaschen. Die Methylenchloridphase wird über Magnesiumsulfat getrocknet und bei 20 Torr und 40° eingedampft. Der Rückstand wird auf 80 g Kieselgel (Merck Si60, 40-63 μm) chromatographiert, wobei mit Ethylacetat eluiert wird. Die Fraktionen, die das Produkt enthalten werden bei 20 Torr und 40° eingedampft. Auf diese Weise werden 2,2 g der Titelverbindung als weisser amorpher Feststoff erhalten. $^1$H-NMR (400 MHz, DMSO-$d_6$): 8.76 (s,b, 1H); 8.00 (d, 1H); 7.56 (m, 2H); 7.44 (m, 4H); 7.21 (d, 1H); 7.16 (d,d, 1H); 7.08 (d, 1H); 6.91 (s, 1H); 5.07 (m, 1H); 3.94 (s, 2H); 3.88 (s, 3H); 3.66 (s, 3H); 2.33 (t, 2H); 1.90-1.53 (m, 8H); 1.47 (m, 2H); 0.89 (t, 3H).

Beispiel 26:

Eine Suspension von 1,38 g N-[4-[5-(Cyclopentyloxycarbonyl-amino)-1-methyl-indol-3-yl-methyl]-3-methoxy-benzoyl]-2-jodbenzolsulfonamid und 1,0 ml Triethylamin in 0,9 ml Dimethylformamid wird unter Rühren bei Raumtemperatur mit 20 mg Kupfer (I)-jodid, 40 mg Bis-(triphenylphosphin)-palladium(II)-dichlorid und 0,24 ml 1-Pentin versetzt. Die Mischung wird 15 Stunden bei 70° gerührt, nochmals mit 0,4 ml 1-Pentin versetzt und weitere 4 Stunden bei 70°C gerührt. Man kühlt ab, setzt 40 ml Ethylacetat und 20 ml Wasser zu, schüttelt, trennt die wässrige Phase ab, extrahiert sie mit 10 ml Ethylacetal und wäscht die vereinigten organischen Phasen mit 20 ml gesättigter Kochsalzlösung. Die organische Phase wird über Magnetiumsulfat getrocknet und unter 11 Torr bei 40° zum Trocknen eingedampft. Der Rückstand wird an 100 g Silicagel chromatographiert. Die Fraktionen 1-3, eluiert mit je 60 ml Ethylacetat, werden verworfen. Die Fraktionen 4-8, eluiert mit demselben Lösungsmittel, werden vereinigt und unter 11 Torr bei 40° eingedampft. Der Rückstand wird nochmals an 80 g Silicagel chromatographiert, wobei wieder mit Ethylacetat eluiert wird. Die Fraktionen, die das Produkt enthalten, werden unter 11 Torr bei 40° eingedampft. Das N-[4-[5-(Cyclopentyl-oxycarbonyl-amino-1-methyl-indol-3-yl-methyl]-3-methoxy-benzoyl]-2-(pent-1-in-1-yl-benzolsulfonamid liegt als beiges Pulver vor. $^1$H-NMR (400 MHz, DMSO-$d_6$): 8.76 (s,b, 1H); 8.00 (d, 1H); 7.56 (m, 2H); 7.44 (m, 4H); 7.21 (d, 1H); 7.16 (d,d, 1H); 7.08 (d, 1H); 6.91 (s, 1H); 5.07 (m, 1H); 3.94 (s, 2H); 3.88 (s, 3H); 3.66 (s, 3H); 2.33 (t, 2H); 1.90-1.53 (m, 8H); 1.47 (m, 2H); 0.89 (t, 3H).

Die Ausgangsmaterialien für dieses Beispiel können wie folgt hergestellt werden:

(a) Zu einer Lösung von 0,79 g 2-Iodbenzolsulfonamid, 0,68 g N-[3-Dimethylamino-propyl]-N′-ethyl-carbodiimid-hydorchlorid und 0,43 g 4-Dimethylaminopyridin in 30 ml Dichlormethan wird unter Rühren in einer Argonatmosphäre eine weisse Lösung von 1,0 g 4-(1-Methyl-5-nitro-indol-3-yl-methyl)-3-methoxy-benzoesäure in 50 ml Tetrahydrofuran zugetropft. Die Mischung wird 15 Stunden bei Raumtemperatur gerührt. Die ausgeschiedenen gelben Kristalle werden abfiltriert, mit 40 ml Ether nachgewaschen und an 150 g Silicagel chromatographiert. Fraktion 1, eluiert mit 80 ml Chloroform-Methanol (9:1), wird verworfen. Die Fraktionen 2-7, eluiert mit demselben Lösungsmittelgemisch, werden vereinigt und unter 11 Torr bei 40° eingedampft. Der Rückstand wird mit 30 ml Ether verrührt. Man filtriert die entstandene Suspension

ab und trocknet die gelben Kristalle 20 Stunden unter 0,3 Torr bei 40°. Das N-[4-(1-Methyl-5-nitro-indol-3-yl-methyl)-3-methoxy-benzoyl]-2-jodbenzolsulfonamid schmilzt bei 179-230° (Zers.).

(b) Eine Lösung von 1,0 g N-[4-(1-Methyl-5-nitro-indol-3-yl-methyl)-3-methoxy-benzoyl]-2-jodbenzolsufonamid in 20 ml Teterahydrofuran wird nach Zusatz von 0,1 g Rhodium auf Kohle (5 %)-Katalysator 4 Stunden bei 20-22° unter Normaldruck hydriert. Es werden nochmals 0,5 g Katalysator zugesetzt und weitere 10 Stunden hydriert. Zur Abtrennung des Katalysators wird das Gemisch durch einen Glasfaserfilter filiert. Man wäscht mit 20 ml Teterahydrofuran nach und engt das Filtrat unter 11 Torr bei 40° zur Trockene ein. Den Rückstand verreibt man mit 30 ml Ether und filtriert die gebildete Suspension ab. Das N-[4-(1-Methyl-5-amino-indol-3-yl-methyl)-3-methoxy-benzoyl]-2-jodbenzolsulfonamid liegt beiges Pulver vor. $^1$H-NMR (400 MHz, DMSO-$d_6$): 8.08 (d,d, 1H); 7.88 (d,d, 1H); 7.55 (d, 1H); 7.42 (t,d, 1H); 7.40 (d,d, 1H); 7.11 (d, 1H); 7.06 (t,d, 1H); 6.93 (d, 1H); 6.88 s, 1H); 6.68 (d, 1H); 6.59 (d,d, 1H); 3.85 s, 2H); 3.84 (s, 3H,-$OCH_3$); 6.36 (3, 3H,-$NCH_3$).

(c) Zu einer Suspension von 0,89 g N-[4-(1-Methyl-5-amino-indol-3-yl-methyl)-3-methoxybenzoyl]-2-jodbenzolsulfonamid und 0,5 ml N-Methylmorpholin in 6 ml Dichlormethan wird unter Argon eine Lösung von 0,23 g Chlorameisensäurecyclopentylester in 4 ml Dichlormethan unter Rühren zugesetzt. Die braune Lösung wird 2 Stunden bei Raumtemperatur gerührt und auf 20 ml 1-n. Salzsäure gegossen. Die wässrige Phase wird abgetrennt und zweimal mit je 10 ml Dichlormethan extrahiert. Die vereinigten organischen Phasen werden mit 10 ml Wasser gewaschen, über Magnesiumsulfat getrocknet und unter 11 Torr bei 40° eingedampft. Der Rückstand wird an 90 g Silicagel flash-chromatographiert. Die Fraktion 1, eluiert mit 40 ml Ethylacetat, wird verworfen. Die Fraktionen 2-4, eluiert mit je 40 ml Ethylacetat, werden vereinigt und unter 11 Törr bei 40° eingedampft. Der Rückstand wird mit 10 ml Ethylacetat-Ether (1:2) aufgerührt. Die Suspension wird abfiltriert und 15 Stunden unter 0,1 Torr bei 40° getrocknet. Das N-[4-[5-(Cyclopentyloxycarbonyl-amino)-1-methyl-indol-3-yl-methyl]-3-methoxy-benzoyl]-2-jodbenzolsulfonamid liegt als beige Krislalle vor. Smp. 200-220° unter Zersetzung. $^1$H-NMR (400 MHz, DMSO-$d_6$): 8.75 (s,b,1H); 8.09 (d,d, 1H); 7.91 (d,d, 1H); 7.60 (d, 1H); 7.57 (d, 1H); 7.44 (d,d, 1H); 7.41 (t,d, 1H); 7.20(d, 1H); 7.17 (d,d, 1H); 7.06 t,d, 1H); 7.02 (d, 1H); 6.90 (s, 1H); 5.08 (m, 1H); 3.92 (s, 2H); 3.85 (s, 3H); 3.66 (s, 3H); 1.90-1.5 (m, 8H).

Beispiel 27:

In analoger Weise wie in einem der vorstehenden Beispiele beschrieben kann man herstellen:

(a) N-[4-[5-(Cyclopentyloxycarbonyl-amino)-1-methyl-indol-3-yl-methyl]-3-methoxy-benzoyl]-2-(hex-1-in-1-yl)-benzolsulfonamid;

(b) N-[4-[5-(Cyclopentyloxycarbonyl-amino)-1-methyl-indol-3-yl-methyl]-3-methoxybenzoyl]-2-(oct-1-in-1-yl)-benzolsulfonamid; weisser Feststoff; $^1$H-NMR (400 MHz, DMSO-$d_6$): 8,89 (s, 1H); 8,03 (d, 1H); 7,59 (d, 1H); 7,58 (d, 1H); 7,55-7,46 (m, 3H); 7,44 (dd, 1H); 7,23 (d, 1H); 7,12 (d, 1H); 7,08 (dd, 1H); 6,95 (s, 1H); 5,07 (m, 1H); 3,95 (s, 2H); 3,92 (s, 3H); 3,68 (s, 3H); 2,39 (t, 2H); 1,92-1,17 (m, 14H); 0,82 (t, 3H);

(c) N-[4-[5-(Cyclopentyloxycarbonyl-amino]-1-methyl-indol-3-yl-methyl]-3-methoxy-benzoyl]-2-(2-cyclohexyl-ethinyl)-benzolsulfonamid;

(d) N-[4-[5-(Cyclopentyloxycarbonyl-amino)-1-methyl-indol-3-yl-methyl]-3-methoxybenzoyl]-2-(2-cyclohex-1-enyl-ethinyl)-benzolsulfonamid; $^1$H-NMR (400 MHz, DMSO-$d_6$): 9,01 (s, b, 1H); 8,07 (m, 1H); 7,60 (m, 4H); 7,54 (d, 1H); 7,41 (dd, 1H); 7,12 (d, 1H); 6,99 (s, 1H); 6,19 (m, 1H); 5,07 (m, 1H); 3,95 (s, 2H); 3,91 (s, 3H); 3,68 (s, 3H); 2,07- 1,47 (m, 16H).

(e) N-[4-[5-(Cyclopentyloxycarbonyl-amino)-1-methyl-indol-3-yl-methyl]-3-methoxy-benzoyl]-2-(3-hydroxy-3-methyl-pent-1-in-1-yl)-benzolsulfonamid; $^1$H-NMR (400 MHz, DMSO-$d_6$): 8,89 (b, 1H); 7,99 (m, 1H); 7,58 (d, 1H); 7,57 (d, 1H); 7,44 (dd, 1H); 7,41 (m, 3H); 7,23 (d, 1H); 7,17 (dd, 1H); 7,04 (d, 1H); 6,90 (s, 1H); 5,08 (m, 1H); 3,93 (s, 2H); 3,87 (s, 3H); 3,67 (s, 3H); 1,92-1,54 (m, 8H); *1,59* (m, 2H); 1,35 (s, 3H); 0,93 (t, 3H).

(f) N-[4-[5-(Cyclopentyloxycarbonyl-amino)-1-methyl-indol-3-yl-methyl]-3-methoxybenzoyl]-2-(3-acetyloxy-3-methyl-pent-1-in-1-yl)-benzolsulfonamid;

(g) N-[4-[5-(Cyclopentyloxycarbonyl-amino)-1-methyl-indol-3-yl-methyl]-3-methoxybenzoyl]-2-(3-hydroxy-3-ethyl-pent-1-in-1-yl)-benzolsulfonamid; $^1$H-NMR (400 MHz, DMSO-$d_6$): 8,88 (b, 1H); 7,99 (m, 1H); 7,58 (d, 1H); 7,57 (d, 1H); 7,43 (dd, 1H); 7,41 (m, 3H); 7,22 (d, 1H); 7,17 (dd, 1H); 7,03 (d, b, 1H); 6,89 (s, 1H); 5,09 (m, 1H); 3,93 (s, 2H); 3,86 (s, 3H); 3,67 (s, 3H); 1,91-1,5 (m, 8H); 1,58 (m, 4H); 0,92 (t, 6H).

(h) N-[4-[5-(Cyclopentyloxycarbonyl-amino)-1-methyl-indol-3-yl-methyl]-3-methoxybenzoyl]-2-(3-hydroxy-4-methyl-pent-1-in-1-yl)-benzolsulfonamid; weisses Pulver; $^1$H-NMR (400 MHz, DMSO-$d_6$): 9,00 (s,b, 1H); 8,01 (d,b, 1H); 7,59 (b, 1H); 7,55 (d, 1H); 7,49 (m,b, 3H); 7,42 (dd, 1H); 7,24 (d, 1H); 7,16 (dd, 1H); 7,05 (d,b, 1H); 6,94 (s, 1H); 5,07 (m, 1H); 4,19 (d,b 1H); 3,93 (s, 2H); 3,88 (s, 3H); 3,67 (s, 3H); 1,90-1,53 (m, 9H); 0,89 und 0,87 (2d, 6H);

(i) N-[4-[5-(Cyclopentyloxycarbonyl-amino)-1-methyl-indol-3-yl-methyl]-3-methoxybenzoyl]-2-(3-acetyloxy-4-methyl-pent-1-in-1-yl)-benzolsulfonamid; weisses Pulver; $^1$H-NMR (400 MHz, DMSO-$d_6$): 8,99 (s,b, 1H); 8,06 (m, 1H); 7,63 (m, 3H); 7,57 (b, 1H); 7,52 (d, 1H); 7,41 (dd, 1H); 7,23 (d, 1H); 7,13 (dd, 1H); 6,98 (s, 1H); 5,42 (d, 1H); 5,05 (m, 1H); 3,90 (s, 3H); 3,84 (s, 2H); 3,66 (s, 3H); 1,97 (m, 1H); 1,95 (s, 3H); 3,66 (s, 3H); 1,97 (m, 1H); 1,95 (s, 3H); 1,90-1,5() (m, 8H); 0,89 und 0,84 (2d, 6H);

(j) N-[4-[5-(Cyclopentyloxycarbonyl-amino)-1-methyl-indol-3-yl-methyl]-3-methoxybenzoyl]-2-[2-(1-hydroxy-cyclohexyl)-ethinyl]-benzolsulfonamid; weisser Feststoff; $^1$H-NMR (400 MHz, DMSO-$d_6$): 8,88 (s, 1H); 8,05 (d, 1H); 7,65-7,50 (m, 5H); 7,42 (dd, 1H); 7,23 (d, 1H); 7,18 (d,d, 1H); 7,11 (d, 1H); 6,97 (s, 1H); 5,08 (m, 1H); 3,97 (s, 3H); 3,92 (s, 3H); 3,68 (s, 3H); 1,90-1,07 (m, 18H).

(k) N-[4-[5-(Cyclopentyloxycarbonyl-amino)-1-methyl-indol-3-yl-methyl]-3-methoxy-benzoyl]-[2-(1-hydroxy-cyclopentyl)-ethinyl] -benzolsulfonamid; weisses Pulver; $^1$H-NMR (400 MHz; DMSO-$d_6$): 8,85 (s, 1H); 7,97 (m, 1H); 7,58 (m, 2H); 7,42 (dd, 1H); 7,32 (s, b, 3H); 7,20 (d, 1H); 7,18 (dd, 1H); 6,99 (d, 1H); 6,84 (s, 1H); 5,05 (m, 1H); 3,90 (s, 3H); 3,82 (s, 3H); 3,63 (s, 3H); 1,98-1,50 (m, 16H); und

(l) N-[4-[5-(Cyclopentyloxycarbonyl-amino)-1-methyl-indol-3-yl-methyl]-3-methoxy-benzoyl]-[2-(1-hydroxy-cyclopropyl)-ethinyl]-benzolsulfonammid.

Beispiele A bis H:

Pharmazeutische Präparate.

Unter dem Ausdruck "Wirkstoff" ist nachstehend eine Verbindung I, in freier Form oder in Form eines pharmazeutisch verwendbaren Salzes, zu verstehen, insbesondere eine solche Verbindung, die als Produkt in den Beispielen 1 bis 27 beschrieben ist, z.B. das N-[4-[5-(Cyclopentyloxycarbonyl-amino)-1-methylindol-3-yl-methyl]-3-methoxy-benzoyl]-2-(5-methoxypent-1-in-1-yl)-benzolsulfonamid.

Beispiel A:

Eine treibmittelhaltige, feststoffaerosolbildende Inhalationssuspension, enthaltend 0,1 Gewichtsprozent Wirkstoff.

| Zusammensetzung | Gewichtsprozent |
|---|---|
| Wirkstoff, mikronisiert | 0,1 |
| Sorbitantrioleat | 0,5 |
| Treibmittel A (Trichlortrifluorethan) | 4,4 |
| Treibmittel B (Dichlordifluormethan und | 15,0 |
| 1,2-Dichlortetrafluorethan) | 80,0 |

Der Wirkstoff wird unter Feuchtigkeitsausschluss mit Hilfe eines üblichen Homogenisators unter Zusatz des Sorbitantrioleats im Trichlortrifluorethan suspendiert und die Suspension in einen mit einem Dosierventil versehenen Aerosolbehälter abgefüllt. Der Behälter wird verschlossen und unter Druck mit dem Treibmittel B aufgefüllt.

Beispiel B:

Eine zur Inhalation geeignete, etwa zweiprozentige, wässrige Lösung des Wirkstoffs in Form seines Natrium- oder Kalium-salzes.

| Zusammensetzung | |
|---|---|
| Wirkstoff (K- oder Na-Salz) | 2000 mg |
| Ethylendiamintetraessigsäure-Dinatriumsalz | 10 mg |
| Benzalkoniumchlorid | 10 mg |
| Wasser, frisch destilliert | ad 100 ml |
| Treibmittel | nach Bedarf |

Der Wirkstoff wird in etwa 60 ml frisch destilliertem Wasser gelöst und der Stabilisator (Ethylendiamintetraessigsäure-Dinatriumsalz) und das Konservierungsmittel (Benzalkoniumchlorid) werden hinzugegeben. Nach vollständiger Auflösung aller Komponenten wird die erhaltene Lösung auf 100 ml aufgefüllt und in Druckfläschchen abgefüllt. Die Fläschchen werden gasdicht verschlossen. Das Treibmittel wird nach Bedarf gasförmig unter Druck oder in flüssiger Form zugegeben.

Beispiel C:

Eine Salbe, enthaltend 0,05 Gewichtsprozent Wirkstoff.

| Zusammensetzung | Gewichtsprozent |
|---|---|
| Wirkstoff | 0,05 |
| Vaseline | 45,00 |
| Praffinöl | 19,60 |
| Cetylalkohol | 5,00 |
| Bienenwachs | 5,00 |
| Sorbitan-sesquioleat | 5,00 |
| p-Hydroxybenzoesäureester | 0,20 |
| Wasser, entmineralisiert | 20,15 |

Die Fettstoffe und Emulgatoren werden zusammengeschmolzen. Das Konservierungsmittel wird in Wasser gelöst und die Lösung in die Fettschmelze bei erhöhter Temperatur einemulgiert. Nach dem Erkalten wird eine Suspension des Wirkstotfs in einem Teil der Fettschmelze in die Emulsion eingearbeitet.

Beispiel D:

Augentropfen, enthaltend 0,3 Gewichtsprozent Wirkstoff.

| Zusammensetzung (10000 Flaschen à 10 ml) | Gewichtsprozent |
|---|---|
| Wirkstoff | 0,30 |
| Dinatriumphosphat | 0,31 |
| Zitronensäure | 0,15 |
| Natriumchlorid | 0,35 |
| Natriumpyrosulfit | 0,10 |
| Benzalkoniumchlorid | 0,01 |
| Wasser, entmineralisiert | 98,78 |

Der Wirkstoff und alle angegebenen Zusatzstoffe werden unter einer Stickstoffatmosphäre in 80 l entmineralisiertes Wasser eingerührt. Nach der vollständigen Auflösung aller Bestandteile wird die Lösung mit entmineralisiertem Wasser auf 1001 aufgefüllt, in einem Autoklaven bei 120° während 20 Minuten sterilisiert und anschliessend unter sterilen Bedingungen durch ein Membranfilter (Porendurchmesser: 0,2 µm) filtriert. Je 10 ml des Filtrats werden unter aseptischen Bedingungen in eine Flasche mit Tropfpipettenverschluss abgefüllt.

Beispiel E:

Tabletten, enthaltend je 50 mg Wirkstoff.

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g der Kartoffelstärke vermischt und die Mischung mit einer ethanolischen Lösung der Gelatine befeuchtet und durch ein Sieb granuliert. Nach dem Trocknen mischt man den Rest der Kartoffelstärke, das Magnesiumstearat, den Talk und das Siliciumdioxid zu und presst das Gemisch zu Tabletten von je 145 mg Gewicht und 50 mg Wirkstoffgehalt, die gewünschtenfalls mit Teilkerben zur feineren Anpassung der Dosierung versehen sein können.

Beispiel F:

Lacktabletten, enthaltend je 100 mg Wirkstoff.

| Zusammensetzung (1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt. Das Gemisch wird mit einem Kleister, hergestellt aus 15 g Maisstärke und Wasser (unter Erwärmen), befeuchtet und granuliert. Das Granulat wird getrocknet und der Rest der Maisstärke, der Talk und das Calciumstearat werden mit dem Granulat vermischt. Das Gemisch wird zu Tabletten (Gewicht: je 280 mg) verpresst und diese werden mit einer Lösung der Hydroxypropylmethylcellulose und des Schellacks in Dichlormethan lackiert (Endgewicht der Lacktabletten: je 283 mg).

Beispiel G:

Gelatinesteckkapseln, enthaltend je 100 mg Wirkstoff.

| Zusammensetzung (1000 Kapseln) | |
|---|---|
| Wirkstoff | 100,0 g |
| Lactose | 250,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoft hinzugesiebt. Beide Komponenten werden innig gemischt. Dann wird zunächst die Lactose durch ein Sieb mit einer Maschenweite von 0,6 mm und anschliessend die mikrokristalline Cellulose durch ein Sieb mit einer Maschenweite von 0,9 mm hinzugesiebt. Dann werden alle vier Komponenten 10 Minuten innig gemischt. Zuletzt wird das Magnesiumstearat durch ein Sieb mit einer Maschenweite von 0,8 mm hinzugesiebt. Nach weiterem Mischen (3 Minuten) werden je 390 mg der erhaltenen Formulierung in Gelatinesteckkapseln der Grösse 0 abgefüllt.

Beispiel H:

Eine Injektions- oder Infusionslösung, enthaltend 5 mg Wirkstoff je 2,5 ml-Ampulle.

| Zusammensetzung (1000 Ampullen) | |
|---|---|
| Wirkstoff | 5,0 g |
| Natriumchlorid | 22,5 g |
| Phosphatpufferlösung (pH: 7,4) | 300,0 g |
| entmineralisiertes Wasser | ad 2500,0 ml |

Der Wirkstoff und das Natriumchlorid werden in 1000 ml entmineralisiertem Wasser gelöst. Die Lösung wird durch ein Mikrofilter filtriert. Man versetzt das Filtrat mit der Phosphatpufferlösung und füllt das Gemisch mit entmineralisiertem Wasser auf 2500 ml auf. Zur Herstellung von Dosiseinheitsformen werden je 2,5 ml des Gemisches in Glasampullen abgefüllt, die dann je 5 mg Wirkstoff enthalten.

## Patentansprüche

**1.** Eine Verbindung der Formel I,

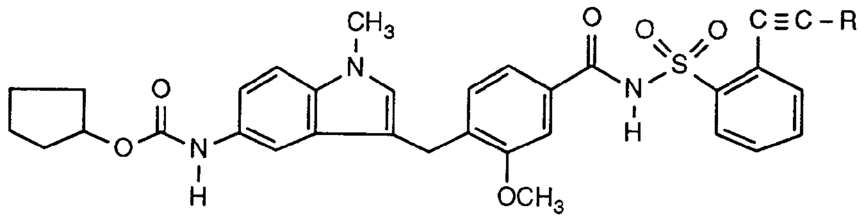

(I)

worin R Wasserstoff oder $C_1$-$C_7$-Alkyl bedeutet oder worin R für das Strukturelement -alk-$R_1$ steht, wobei alk $C_1$-$C_7$-Alkylen, $C_2$-$C_7$-Alkyliden oder $C_3$-$C_6$-Cycloalkyliden bedeutet und $R_1$ Hydroxy, $C_1$-$C_7$-Alkoxy, Phenyl-$C_1$-$C_7$-alkoxy oder $C_2$-$C_7$-Alkanoyloxy bedeutet; in freier Form oder in Form eines Salzes.

**2.** Verbindung gemäss Anspruch 1 der Formel I, worin R Wasserstoff oder $C_1$-$C_5$-Alkyl bedeutet oder worin

R für das Strukturelement -alk-$R_1$ steht, wobei alk $C_1$-$C_5$-Alkylen, $C_2$-$C_7$-Alkyliden oder $C_3$-$C_6$-Cycloalkyliden bedeutet; und $R_1$ Hydroxy, $C_1$-$C_4$-Alkoxy, Phenyl-$C_1$-$C_4$-alkoxy oder $C_2$-$C_7$-Alkanoyloxy bedeutet; in freier Form oder in Form eines Salzes.

**3.** Verbindung gemäss Anspruch 1 der Formel I, worin R Wasserstoff oder $C_1$-$C_4$-Alkyl bedeutet, oder worin R für das Strukturelement -alk-$R_1$ steht, wobei alk $C_1$-$C_4$-Alkylen oder $C_2$-$C_5$-Alkyliden bedeutet, und $R_1$ Hydroxy, $C_1$-$C_4$-Alkoxy oder $C_2$-$C_5$-Alkanoyloxy bedeutet; in freier Form oder in Form eines Salzes.

**4.** Verbindung gemäss Anspruch 1 der Formel I, worin R Wasserstoff oder $C_1$-$C_3$-Alkyl bedeutet; in freier Form oder in Form eines Salzes.

**5.** Verbindung gemäss Anspruch 1 der Formel I, worin R für das Strukturelement -alk-$R_1$ steht; wobei alk $C_1$-$C_4$-Alkylen oder $C_2$-$C_5$-Alkyliden bedeutet und $R_1$ Hydroxy bedeutet; in freier Form oder in Form eines Salzes.

**6.** Verbindung gemäss Anspruch 1 der Formel I, worin R für das Strukturelement -alk-$R_1$ steht; wobei alk 1,3-Propylen, Ethyliden, 2,2-Propyliden bedeutet und $R_1$ Hydroxy bedeutet; in freier Form oder in Form eines Salzes.

**7.** N-[4-[5-(Cyclopentyloxycarbonyl-amino)-1-methyl-indol-3-yl-methyl]-3-methoxy-benzoyl]-2-ethinyl-benzolsulfonamid gemäss Anspruch 1 oder ein pharmazeutisch anwendbares Salz davon.

**8.** N-[4-[5-(Cyclopentyloxycarbonyl-amino)-1-methyl-indol-3-yl-methyl]-3-methoxy-benzoyl]-2-(but-1-in-1-yl)-benzolsulfonamid gemäss Anspruch 1 oder ein pharmazeutisch anwendbares Salz davon.

**9.** N-[4-[5-(Cyclopentyloxycarbonyl-amino)-1-methyl-indol-3-yl-methyl]-3-methoxy-benzoyl]-2-(3-methyl-but-1-in-1-yl)-benzolsulfonamid gemäss Anspruch 1 oder ein pharmazeutisch anwendbares Salz davon.

**10.** N-[4-[5-(Cyclopentyloxycarbonyl-amino)-1-methyl-indol-3-yl-methyl]-3-methoxy-benzoyl]-2-(3-hydroxy-3-methyl-but-1-in-1-yl)-benzolsulfonamid gemäss Anspruch 1 oder ein pharmazeutisch anwendbares Salz davon.

**11.** N-[4-[5-(Cyclopentyloxycarbonyl-amino)-1-methyl-indol-3-yl-methyl]-3-methoxy-benzoyl]-2-(5-hydroxy-pent-1-in-1-yl)-benzolsulfonamid gemäss Anspruch 1 oder ein pharmazeutisch anwendbares Salz davon.

**12.** Ein pharmazeutisches Präparat enthaltend als Wirkstoff eine Verbindung gemäss einem der Ansprüche 1 bis 11, oder ein pharmazeutisch anwendbares Salz davon, und mindestens ein pharmazeutisch verwendbares Trägermaterial.

**13.** Eine Verbindung gemäss einem der Ansprüche 1 bis 11 zur Anwendung in einem Verfahren zur therapeutischen Behandlung des tierischen oder menschlichen Körpers.

**14.** Eine Verbindung gemäss einem der Ansprüche 1 bis 11 zur Verwendung bei der Behandlung von allergischen Zuständen und Erkrankungen.

**15.** Verwendung einer Verbindung gemäss einem der Ansprüche 1 bis 11, in freier Form oder in Form eines pharmazeutisch verwendbaren Salzes, zur Herstellung eines pharmazeutischen Präparates zur Behandlung von allergischen Zuständen und Erkrankungen.

**16.** Verfahren zur Herstellung einer Verbindung der Formel I gemäss Anspruch 1, in freier Form oder in Form eines Salzes, dadurch gekennzeichnet, dass man

a) eine Verbindung der Formel IIa,

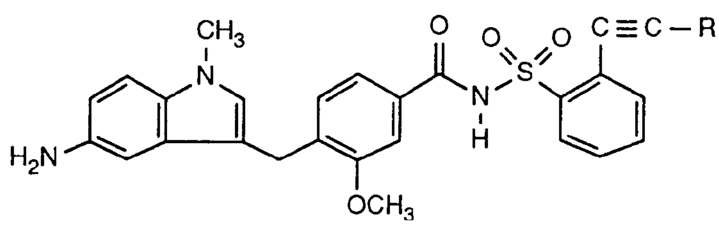

(IIa)

oder ein Salz davon, mit einer Verbindung der Formel IIb,

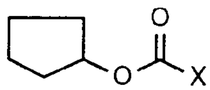

(IIb)

worin X eine nucleofuge Abgangsgruppe ist, unter Abspaltung einer Verbindung H-X umsetzt; oder
b) in einer Verbindung der Formel III,

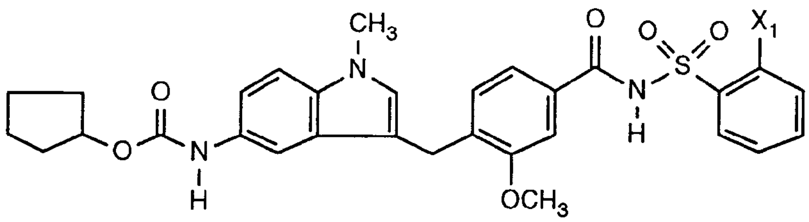

(III)

worin $X_1$ eine in den Rest -C≡C-R überführbare Gruppe bedeutet, oder einem Salz davon, $X_1$ in den Rest -C≡C-R überführt; oder
c) eine Verbindung der Formel IVa,

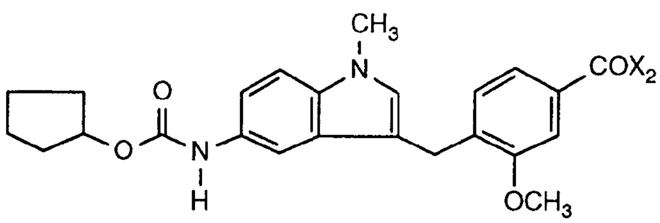

(IVa)

worin $X_2$ für

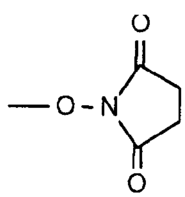

(IVa'),

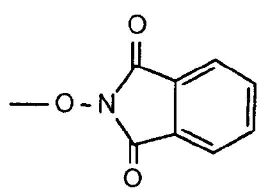

(IVa'')

oder

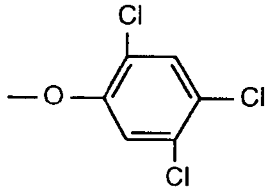

(IVa''')

steht, mit einer Verbindung der Formel IVb,

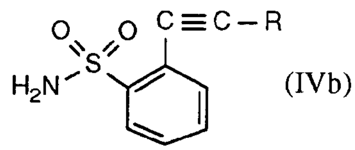

(IVb)

oder einem Salz davon, umsetzt;
und gewünschtenfalls jeweils eine Verbindung der Formel I in eine andere Verbindung der Fomel I überführt und/oder eine verfahrensgemäss erhältliche freie Verbindung I in ein Salz überführt oder ein verfahrensgemäss erhältliches Salz einer Verbindung I in die freie Verbindung I oder in ein anderes Salz umwandelt und/oder die Verbindung der Formel I oder ein Salz davon isoliert.

Europäisches Patentamt

# EUROPÄISCHER RECHERCHENBERICHT

Nummer der Anmeldung

EP 92 81 0795

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 199 543 (ICI AMERICAS INC.)<br>* Spalte 1, Zeile 1-13; Beispiel 105 *<br>--- | 1,12,15 | C07D209/24<br>C07D209/18<br>A61K31/40 |
| A | EP-A-0 337 766 (ICI AMERICAS INC.)<br>* Ansprüche *<br>----- | 1,12,15 | |
| | | | RECHERCHIERTE SACHGEBIETE (Int. Cl.5 ) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 25 NOVEMBER 1992 | VAN BIJLEN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)